## Europäisches Patentamt

## European Patent Office

## Office européen des brevets

(11) Publication number: **0 118 393**
**B1**

# EUROPEAN PATENT SPECIFICATION

(45) Date of publication of patent specification: 03.05.89

(51) Int. Cl.⁴: **C 12 N 15/00,** C 12 N 5/00, C 12 P 21/00

(21) Application number: 84810066.5

(22) Date of filing: 06.02.84

(54) Methods and compositions for expression of competent eukaryotic gene products.

(30) Priority: 07.02.83 US 464232
17.06.83 US 504593
05.01.84 US 568176

(43) Date of publication of application:
12.09.84 Bulletin 84/37

(45) Publication of the grant of the patent:
03.05.89 Bulletin 89/18

(84) Designated Contracting States:
AT BE CH DE FR GB IT LI LU NL SE

(56) References cited:
EP-A-0 045 573
EP-A-0 060 057
EP-A-0 068 740

SCHLESINGER, ASHBURGER, TISSIERES "Heat Shock from Bacteria to Man", 1982, HARBOUR PRESS, Cold Spring, pp. 43-48

CELL, vol. 30, no. 2, September 1982, Cambridge, Mass., H.R.B. PELHAM "A regulatory upstream promoter element in the drosophila Hsp 70 heat-shock gene", pp. 517-528

(73) Proprietor: BATTELLE MEMORIAL INSTITUTE
7 route de Drize
CH-1227 Carouge/Genève (CH)

(72) Inventor: Bromley, Peter
113, rue de Genève
CH-1226 Thonex (CH)
Inventor: Voellmy, Richard
c/o M. Schilter 34 Kornhausstrasse
CH-8006 Zurich (CH)

(74) Representative: Dousse, Blasco et al
7, route de Drize
CH-1227 Carouge/Genève (CH)

(56) References cited:
SCHLESINGER, ASHBURGER, TISSIERES "Heat shock from bacteria to man", 1982, HARBOUR PRESS, Cold Spring, pp. 27-34

Courier Press, Leamington Spa, England.

# EP 0 118 393 B1

## Description

### Background of the invention

#### 1. Field of the invention

The industrial production of proteins coded by specific genes such as those encoding hormones, clotting factors, virus proteins, insulin, interferon and others, involves selecting and isolating gene sequences from viral, eukaryotic and other RNA or DNA, splicing of the sequences in the form of DNA into DNA vectors to provide recombinant DNA, and introducing said recombinant DNA into host cells capable of expressing these genes. The vectors may impart to the transformed host cells a phenotypic trait used for isolation and cloning purposes. The gene products are isolated from cell cultures by usual techniques.

Up to now, efforts in this field have usually been based on the adaptation of microorganisms as host cells for expressing genes of interest. Indeed, bacteria are often the organisms of choice since they can be grown rapidly, in large quantities and at low cost. Foreign DNA can be introduced easily into bacterial cells by using vectors such as plasmids, cosmids, viruses and the like.

Use of bacterial hosts, however, is not always sufficient to obtain expression of mature eukaryotic proteins. Many important eukaryotic proteins are modified by glycosylation, acetylation, phosphorylation, specific proteolytic cleavage and other forms of processing. In many instances post-transcriptional and post-translational modifications are crucial in determining the final biological properties of protein products. Non-proteolytic post-translational modifications of certain proteins such as glycosylation, acetylation, phosphorylation and others may not occur correctly, if at all, in bacteria or cell types significantly different from the cell type in which the gene product of interest is normally produced in the organism. The correct form of these modifications may prove critical in the synthesis of fully competent gene products by molecular cloning techniques. For example, when the gene product is a glycoprotein, such as the haemagglutinin of influenza virus, the precise nature of glycosylation may influence the efficiency of antibodies raised against this synthetic protein to protect human beings or animals against influenza virus infections. Moreover, when glycosylation, acetylation or phosphorylation or other modifications are required to stabilize, activate, or mediate intracellular transport or excretion of a protein, the precision of these modifications may be critical to the utility of these genetically engineered protein products in practice.

Because of the above-mentioned shortcomings of using bacteria for the synthesis of complex gene products, there have been a numer of attempts to introduce DNA encoding specific eukaryotic proteins into eukaryotic cells. DNA can be introduced by co-transformation into suitable mutant cells which are deficient in the production of a particular enzyme, such as thymidine kinase or hypoxanthine phosphoribosyl transferase. Then by culturing the cells in a selective medium deficient in the enzyme product, transformed cells may be selected for by their ability to grow, i.e., their ability to produce the enzyme. Alternatively, cells can be co-transfected in a positive sense to add a gene that will transduce cells to become selectively resistant to a drum or selective medium such methotrexate, neomycin, or others. Although many of these attempts have enjoyed some measure of success, in most cases the yeild of mature gene products has been quite limited.

In order to maximize the yields of expression of fully competent gene products of interest, it would be desirable that the host expression unit system employed consists of an expression vector derived from one cell type or organism that is capable of synthesizing the said fully competent gene products, and that this expression vector directs the synthesis of said gene products in the same or similar cell type or organism as host.

#### 2. Description of the prior art

The heat-shock phenomenon has been studied most extensively in *Drosophila melanogaster*. For a review, see Ashburner and Bonner (1979) Cell *17*: 241—254. When *Drosophila* cells or organs, normally at about 25°C, are exposed to a heat treatment at 35—37°C, a family of heat-shock genes is activated and most of the genes active at 25°C are no longer transcribed. Seven genes code for polypeptides with molecular weights between 22,000 and 84,000 daltons. During heat treatment these heat-shock polypeptides are synthesized almost exclusively and after 8 hrs, represent 10% of the total cellular protein (Arrigo, P. (1979) Ph. D. Thesis, University of Geneva). During heat treatment of *Drosophila* cells, much of the polysome bound mRNA codes for heat-shock proteins (McKenzie *et al.* (1975) *72:* 1117—1121; Mirault *et al.* (1979) Cold Spring Harbor Symp. Quant. Biol. *42:* 819—827).

All seven *Drosophila* heat-shock protein genes have been cloned. See, Livak *et al.* (1978) PNAS *75:* 5613—5617; Schedl *et al.* (1978) Cell *14:* 921—929; Graig *et al.* (1979) Cell *16:* 575—588; Holmgren *et al.* (1979) Cell *18:* 1359—1370; Wadsworth *et al.* (1980) PNAS *77:* 2134—2137; Corces *et al.* (1980) PNAS *77:* 5390—5393; Voellmy *et al.* (1981) Cell *23:* 261—270. A number of the genes have been sequenced. See Karch and Torok (1980) Nucleic Acid Res. *8:* 3105—3123, and Ingola and Craig (1982) PNAS *79:* 2360—2364.

All eukaryotic organisms appear to posses heat shock genes. See Kelly and Schlesinger (1978) Cell *15:* 1277—1288. Many of the heat shock genes appear to be conserved throughout widely diverse species, and *Drosophila* heat shock genes have been shown to be transcribed in mouse cells (Corces *et al.* (1981) PNAS *78:* 7038—7042), frog cells (Voellmy and Rungger (1982) PNAS *79:* 1776—1780), and monkey cells (Pelham (1982) Cell·*30:* 517—528). Fusion genes consisting of *Drosophila* heat-shock gene regions and Herpes

2

Simplex virus thymidine kinase gene regions are also transcribed in these heterologous cell systems. No evidence has been presented which would suggest that the protein products of these genes are formed. Pelham, H. and Bienz, M. (1982) p. 43—48 in Heat Shock from Bacteria to Man. Ed. Schlesinger, Ashburner and Tissières. Cold Spring Harbour Press. Corces *et al.* (1982) p. 27—34 in Heat Shock from Bacteria to Man. Ed. Schlesinger, Ashburner and Tissières. Cold Spring Harbour Press.

## Summary of the invention

DNA constructions are provided comprising control elements derived from heat-shock genes associated with genes of interest, which permit the expression of the gene of interest in both procaryotic and eucaryotic cells. In particular, for the efficient synthesis of fully competent gene products, a homologous combination of expression unit and host cell for expression as defined in claim 1 is used and the nature of this homologous system is that of the same or similar cell type or organism that normally produces the said gene product. Expression vectors suitable for achieving the objects of claim 1 are defined in subsequent claims which also cover a method for the production of proteins of interest by the induced expression of synthetic genes.

## Brief description of the drawings

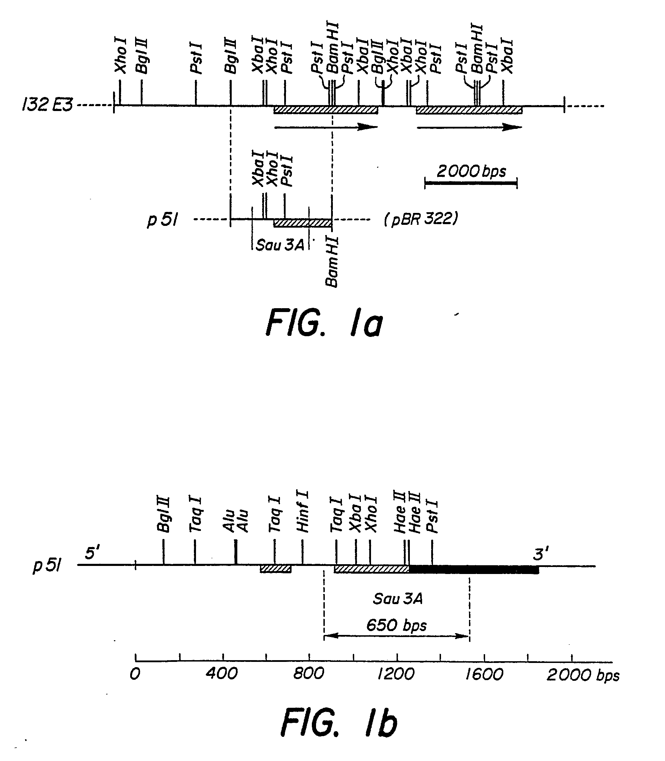

Fig. 1*a* is a schematic representation of a partial restriction map of part of plasmid 132E3 containing two 70 kdal heat-shock protein (hsp) genes (heavy lines) and their orientation (arrows). The lower part of Fig. 1*a* represents the relevant partial restriction map of plasmid 51 for comparison purposes.

Fig. 1*b* represents on an enlarged scale a more detailed restriction map of part of plasmid 51 with additional restriction sites, including the two characteristic *Sau*3A sites.

Fig. 2 represents the complete DNA sequence of one strand of the DNA preceding the 70 kdal hsp gene in plasmid 51 except for the white sequences on a black background, which are present in another 70 kdal gene sequence, as determined by the procedure of Maxam and Gilbert (1977), PNSA *74*: 560), and in addition some important restriction sequences as well as the site of the start of transcription and translation (arrows).

Figs. 3*a*—3*d* represent a diagrammatic representation of the procedure for the construction of plasmid pRV15.

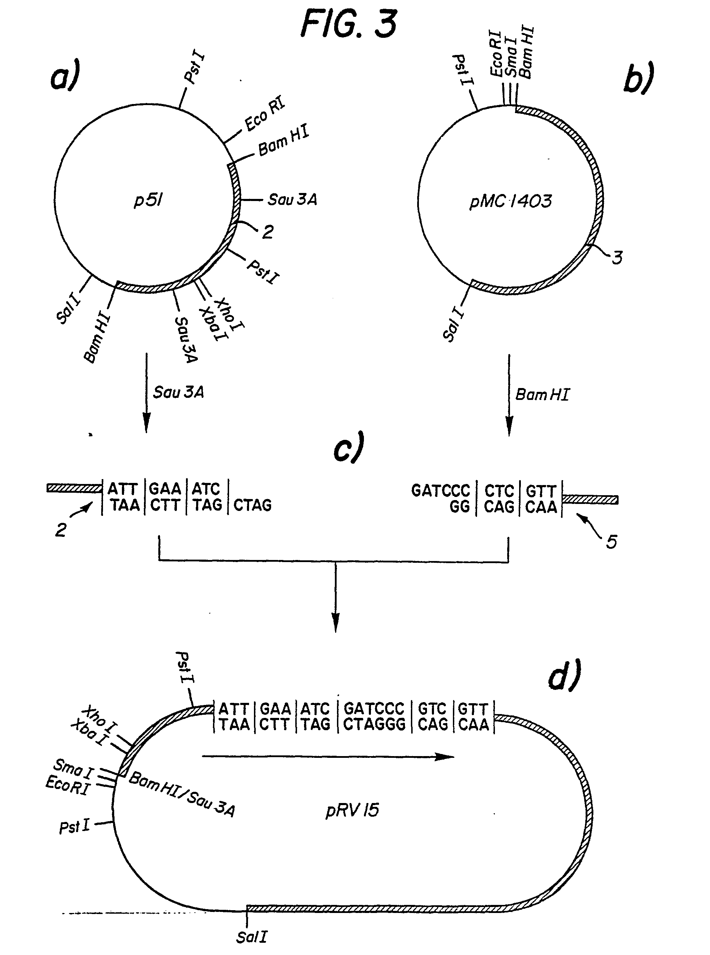

In particular, Fig. 3*a* shows a restriction map of plasmid 51 which after digestion with *Sau*3A gives rise to an approximately 650 bp fragment referred to henceforth as the 650 bp fragment, one end of which is indicated in detail in Fig. 3*c* (left part).

Fig. 3*b* is a restriction map of plasmid pMC1403 which, after restriction with *Bam*HI, leaves one end with the structure indicated in detail in Fig. 3*c* (right part).

Fig. 3*c* represents details of the sequence of the ends produced by the restriction digests of plasmids 51 and pMC1403.

Fig. 3*d* represents the structure of plasmid pRV15 showing the linkage of the *Sau*3A fragment of plasmid 51 in the *Bam*HI site of plasmid pMC1403. The position of the β-galactosidase gene and of the *Drosophila* DNA are indicated in the plasmid by heavy lines. Some restriction sites are also indicated.

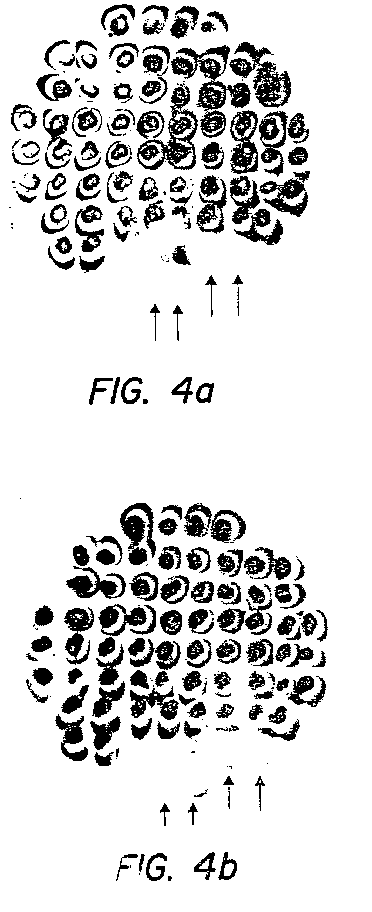

Fig. 4*a* is a photograph showing characterization of the structure of plasmid pRV15 indicated in Fig. 3*d* by the colony hybridization assay of Grunstein and Hogness (1975) PNAS *72*: 3961. This was performed using radioactive probes prepared by nick translation (Maniatis et al., (1975) PNAS *72*: 1185) of either a 2 kbp *Xba*I fragment excised from plasmid 132E3 (see Fig. 1*a*) or a labelled 650 bp *Sau*3A fragment from plasmid 51 (Fig. 4*b*). A number of transformants produed using the construction scheme of Fig. 3 were tested as were some colonies of pMC1403 as a control; these colonies are indicated by arrows in Fig. 4*a*.

Fig. 4*b* represents a similar colony hybridization assay but using the *Sau*3A kbp fragment from plasmid 51 as a probe. Control colonies of pMC1403 are indicated by arrows.

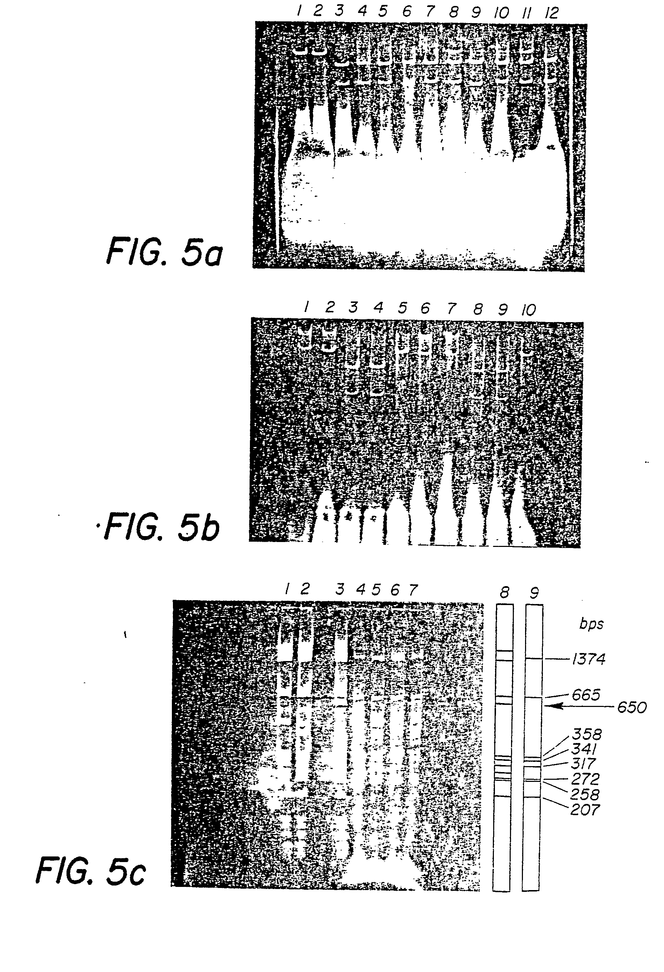

Fig. 5 represents the characterization, by restriction analysis, of the structure for plasmid pRV15 indicated in Fig. 3*d*. Fragment sized in bps are indicated. In particular:

Fig. 5*a* is a photograph showing the migration of various DNA fragments under electrophoresis. DNA was prepared from mini-plasmid preparations (Davis et al., (1980), Methods of Enzymology, Grossmann and Moldave, eds. 65: 404—414) for plasmid pMC1403, pRV15 and two similar isolates, pRV25 and pRV5. These plasmids were digested with restriction enzymes as indicated below and the fragments of DNA produced were electrophoresed on 0.85% agarose gels at 150v for 4 hours after which bands were visualized using ethidium bromide staining and U.V. photography. Identification of the lanes is as follows:

Lane  1  pMC1403—*Sau*3A/*Xho*I
      2  pMC1403—*Sal*I/*Xba*I
      3  pMC1403—*Sal*I/*Eco*RI
      4  pRV25—*Sal*I/*Xho*I
      5  pRV25—*Sal*I/*Xba*I
      6  pRV25—*Sal*I/*Eco*RI
      7  pRV15—*Sal*I/*Xho*I
      8  pRV15—*Sal*I/*Xba*I
      9  pRV15—*Sal*I/*Eco*RI
    10  pRV5—*Sal*I/*Xho*I
    11  pRV5—*Sal*I/*Xba*I
    12  pRV5—*Sal*I/*Eco*RI

Fig. 5b is similar to Fig. 5a for a series of digests and analyses performed identically. The lanes are as follows:

Lane  1  pRV15—*Xho*I
      2  pRV15—*Xba*I
      3  pRV15—*Sal*I/*Xho*I
      4  pRV15—*Sal*I/*Xba*I
      5  pRV15—*Sal*I
      6  pRV5—*Xho*I
      7  pRV5—*Xba*I
      8  pRV5—*Sal*I/*Xho*I
      9  pRV5—*Sal*I/*Xba*I
    10  pRV5—*Sal*I

Fig. 5c is similar to Figs. 5a and 5b and concerns restriction digests and analyses performed for fragments separated on gels of 5% polyacrylamide. Electrophoresis was at 40v for 14 hours.

Lane 1  51—*Sau*3A/*Xba*I
     2  51—*Sau*3A/*Xho*I
     3  51—*Sau*3A
     4  pMC1403—*Sau*3A
     5  pRV15—*Sau*3A/*Xba*I
     6  pRV15—*Sau*3A/*Xho*I
     7  pRV15—*Sau*3A
     8  Diagammatic representation of 51—*Sau*3A
     9  Diagrammatic representation of pBR322—*Sau*3A

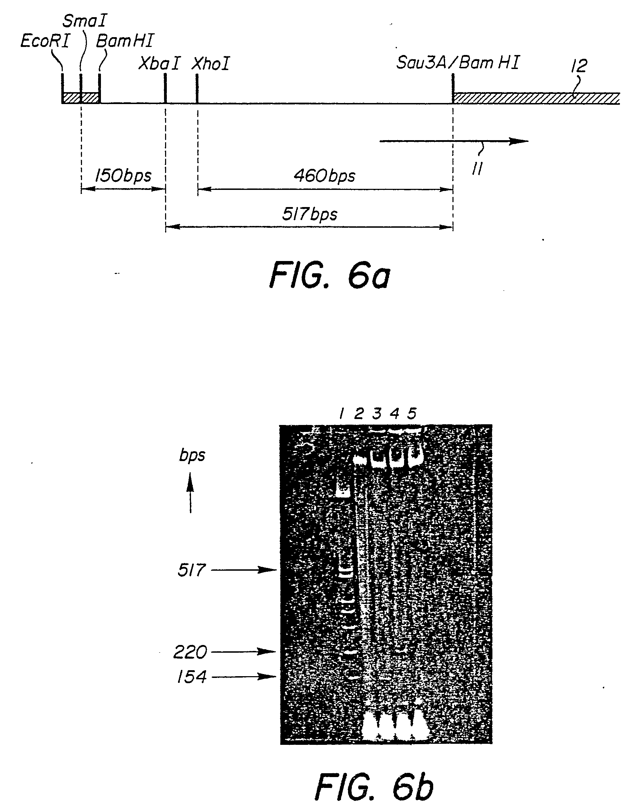

Figs. 6a—6b refer to an analysis of the orientation of the *Drosophila* 70 kdal hsp gene *Sau*3A fragment in plasmid pRV15. In particular:

Fig. 6a is a diagrammatic representation of the sizes of predicted restriction fragments that would result if the desired orientation of *Drosophila* elements and the *E. coli* β-galactosidase gene is indeed the case in plasmid pRV15.

Fig. 6b is a photograph of an acrylamide gel (5%) analysis of the results of restriction of plasmid pRV15 and pBR322 to test the prediction presented in the diagram of Fig. 6a. Analyses were performed as described for the cases represented by Fig. 5c.

Lane 1  pBR322—*Hin*fI
     2  pRV15—*Eco*RI
     3  pRV15—*Xba*I/*Eco*RI
     4  pRV15—*Xho*I/*Eco*RI
     5  pRV15—*Xho*I

The position of bands of sizes 517, 220 and 154 bps are indicated by arrows and are derived from the known sizes of fragments derived by restriction of plasmid pBR322 by *Hin*fI.

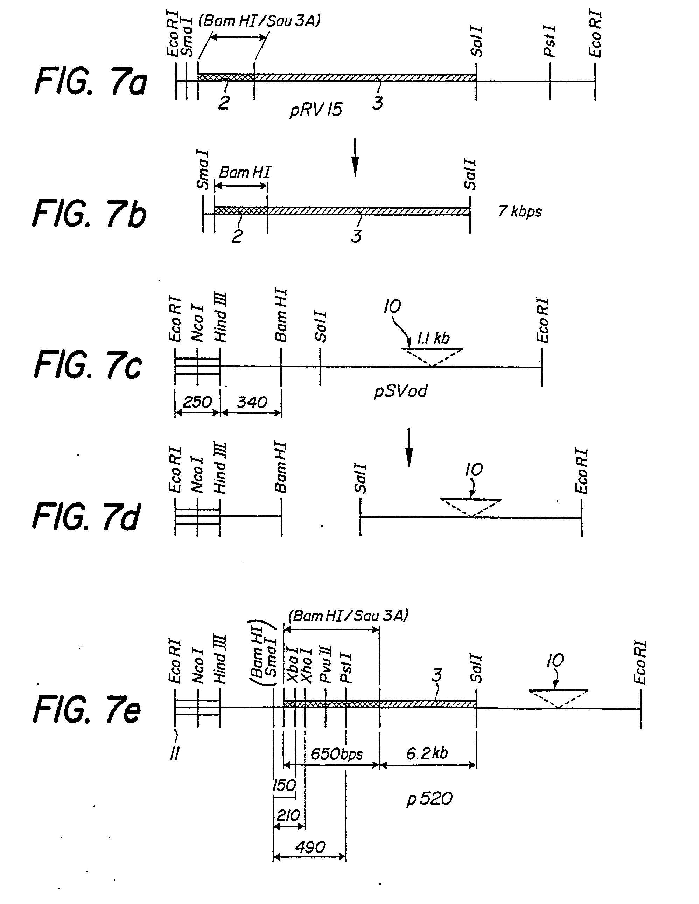

Figs. 7a—7e, which show plasmids or fragments of plasmids after digest with restriction enzymes, represent schematically the construction of plasmid 520, which consists of incorporating the *Sma*I—*Sal*I fragment of pRV15 containing the lac operon fragment and the *Drosophila* 650 bp control element into plasmid pSVod using the *Bam*HI—*Sal*— restriction site. In particular:

Fig. 7a represents pRV15, Fig. 7c represents pSVod; Fig. 7b represents a *Sma*I—*Sal*I fragment of pRV15; Fig. 7d represents pSVod after excision of a *Bam*HI—*Sal*I fragment; and Fig. 7e represents p520.

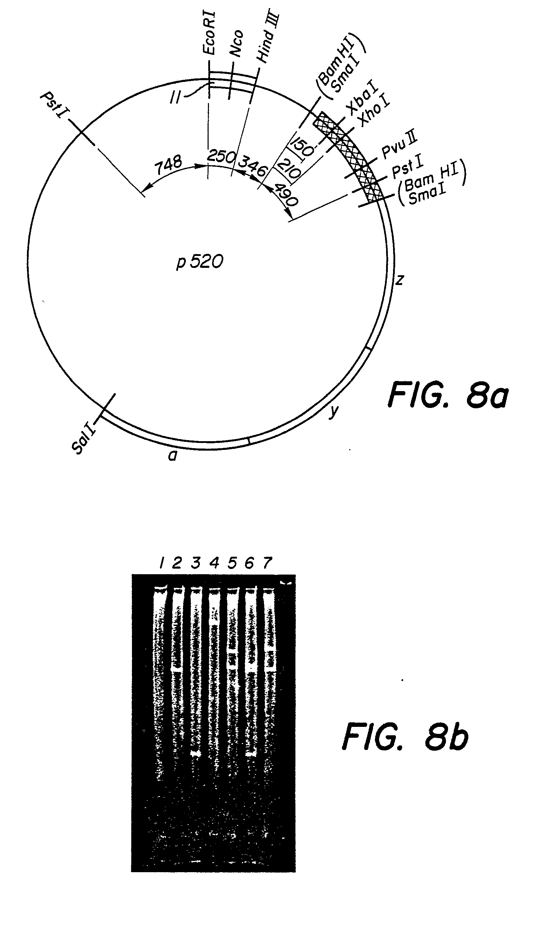

Fig. 8a is a restriction map for plasmid vector 520 indicating the position of selected restriction sites in the structure of the plasmid.

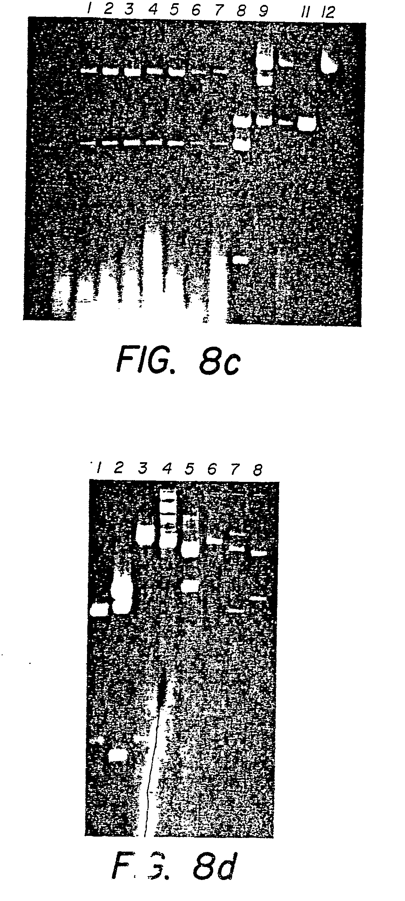

Fig. 8*b* is a photograph of an agarose gel analysis of restriction enzyme digestions of plasmid 520 performed as described in the case of pRV15 (see Fig. 5*a*).

Lane 1—*Hind*III
    2—*Xho*I/*Eco*RI
    3—*Hind*III/*Eco*RI
    4—*Pst*I
    5—*Hind*III/*Pst*I
    6—*Pst*I/*Hind*III/*Eco*RI
    7—*Pst*I/*Eco*RI

Fig. 8*c* is a photograph also referring to the characterization of plasmid 520. It shows agarose gel analysis of various restriction digest of plasmids pSVod, pRV15 and 520-like plasmids, i.e., plasmids 639, 520, 519, X, Y, Z.

Lane 1 639—*Sal*I/*Eco*RI
    2 520—*Sal*I/*Eco*RI
    3 519—*Sal*I/*Eco*RI
    4 516—*Sal*I/*Eco*RI
    5 X—*Sal*I/*Eco*RI
    6 Y—*Sal*I/*Eco*RI
    7 Z—*Sal*I/*Eco*RI
    8 pSVod—*Sal*I/*Eco*RI
    9 pRV15—*Sal*I/EcoRI
   10 pSVod—*Xho*I/*Eco*RI
   11 pRV15—*Xho*I/*Eco*RI

Note that the digests in lanes 8 and 9 are incomplete.

Fig. 8*d* is a photograph showing further restriction digests of plasmid 520 by comparison with digests of plasmids pSVod and pRV15.

Lane 1 pSVod—*Pst*I/*Eco*RI
    2 pSVod—*Hind*III/*Sal*I
    3 pRV15—*Pst*I/*Eco*RI
    4 pRV15—*Hind*III/*Sal*I
    5 pRV15—*Sal*I/*Xba*I
    6 520—*Pst*I/*Eco*RI
    7 520—*Hind*III/*Sal*I
    8 520—*Sal*I/*Xba*I

Note that the digests in lanes 2 and 7 are incomplete.

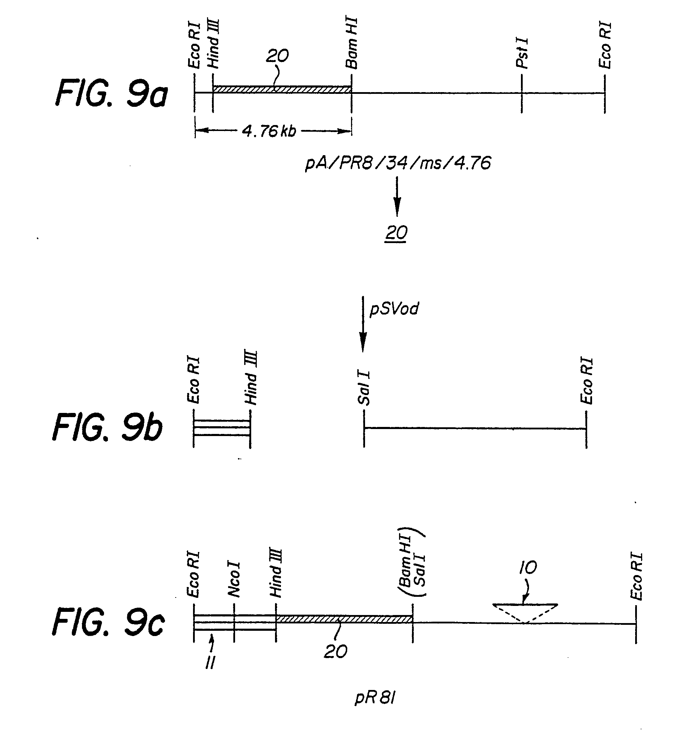

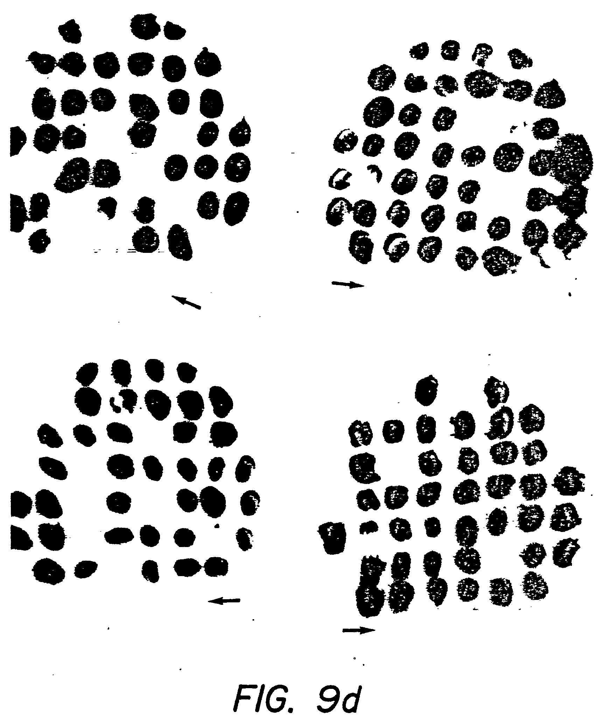

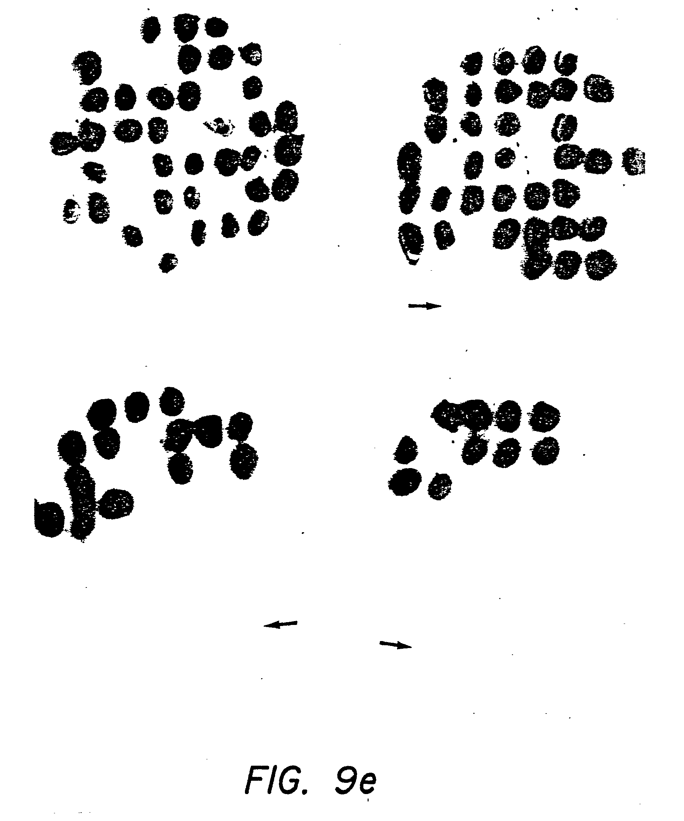

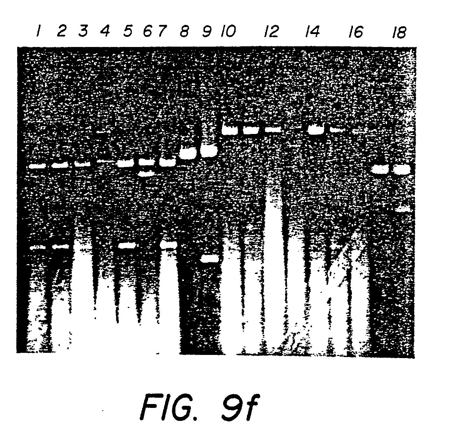

Figs 9*a*—9*c* refer to the construction of plasmid PR81. In particular:

Fig 9*a* represents the Mount Sinai A/PR 8/ms/4.76 plasmid from which a fragment (ref. No. 20) was excised with *Bam*HI and *Hind*III; Fig. 9*b* represents pSVod less a *Hind*III—*Sal*I fragment; and Fig. 9*c* represents plasmid PR81, i.e., the ligation product of the *Bam*HI—*Hind*III fragment 20 with a *Sal*I—*Hind*III digest.

Figs. 9*d* and 9*e* are photographs of a Grunstein colony hybridization analysis of PR81-like plasmids, performed as described for pRV15 (see Fig. 4). The nick-translated probe used was the *Bam*HI/*Hind*III fragment for A/PR 8/ms/4.76. As a control some pSVod containing colonies are indicated by the arrows.

Fig. 9*f* is a photograph of a restriction analysis of PR81-like plasmids shown by colony hybridization analysis in Figs. 9d and 9e.

Lane  1   filter 21, Sample 30—*Eco*RI
     2   filter 21, Sample 44—*Eco*RI
     3   filter 22, Sample 11—*Eco*RI
     4   filter 22, Sample 24—*Eco*RI
     5   filter 23, Sample 10—*Eco*RI
     6   filter 23, Sample 31—*Eco*RI
     7   filter 24, Sample 8—*Eco*RI
     8   pSVod—*Eco*RI
     9   PR 8/ms/4.76—*Eco*RI
   10   filter 21, Sample 30—*Hind*III/*Sa*/I
   11   filter 21, Sample 44—*Hind*III/*Sa*/I
   12   filter 22, Sample 11—*Hind*III/*Sa*/I
   13   filter 22, Sample 24—*Hind*III/*Sa*/I
   14   filter 23, Sample 10—*Hind*III/*Sa*/I
   15   filter 23, Sample 31—*Hind*III/*Sa*/I
   16   filter 24, Sample 8—*Hind*III/*Sa*/I
   17   pSVod—*Hind*III/*Sa*/I
   18   PR 8/ms/4.76—*Hind*III/*Sa*/I

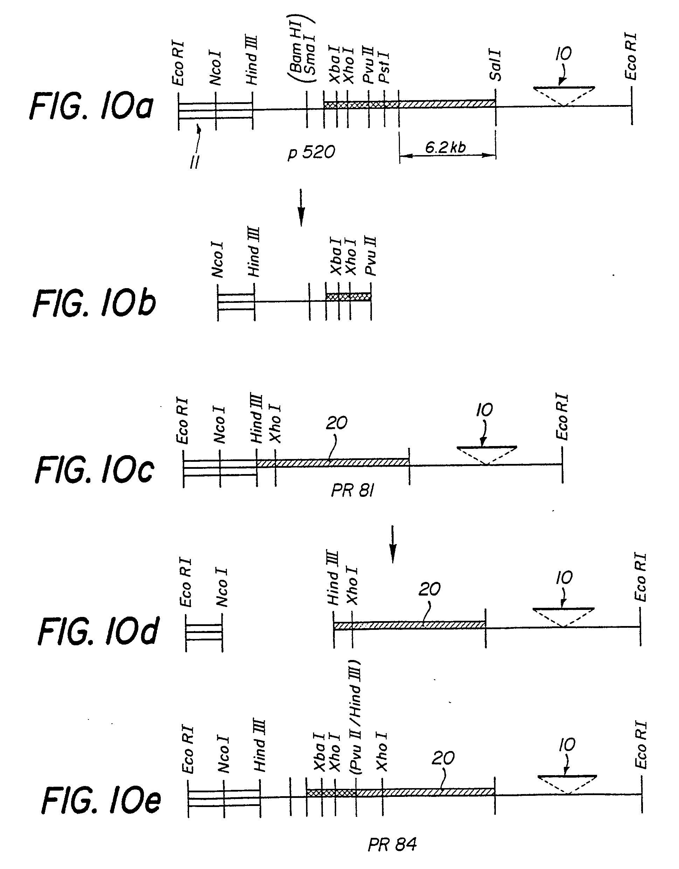

Figs. 10a—10e refer to the construction scheme of plasmid PR84. This plasmid was constructed as shown in the figure and consists of substituting the haemagglutinin (HA) gene 20 present in plasmid PR81 for the β-galactosidase gene in plasmid 520. In particular:

Fig. 10a represents plasmid 520; Fig. 10c represents plasmid PR81; Fig. 10b represents an *Nco*I—*Pvu*II fragment excised from 520; Fig. 10d represents PR81 after digestion with *Hind*III and *Nco*I; Fig. 10e represents schematically PR84.

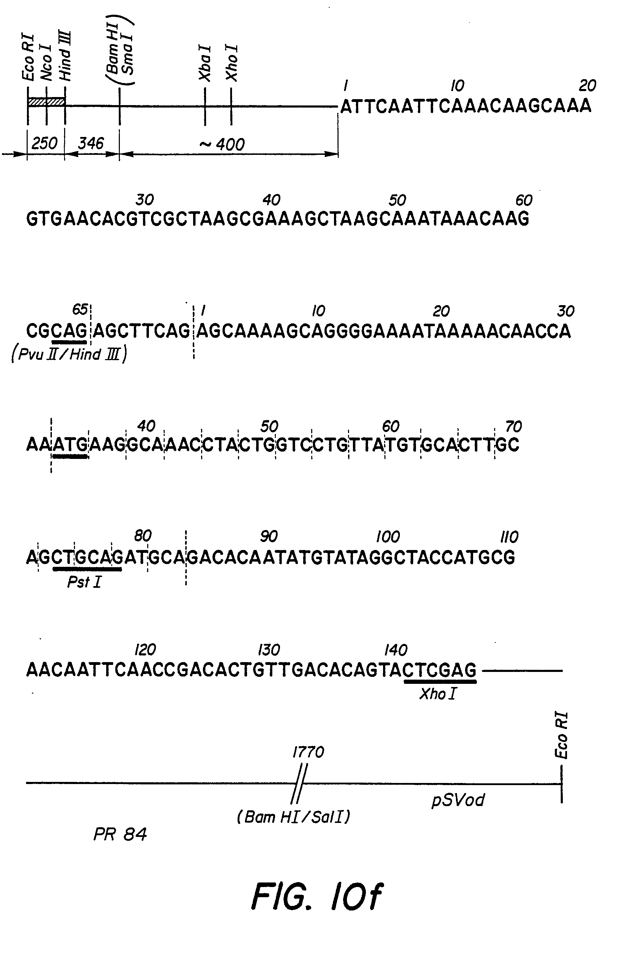

Fig. 10f is a more detailed representation of PR85 with one strand of base-pairs including the sequence originating from PR 8/ms; only the crucial joining region between the *Drosophila* HS control element and the HA start sequence is detailed.

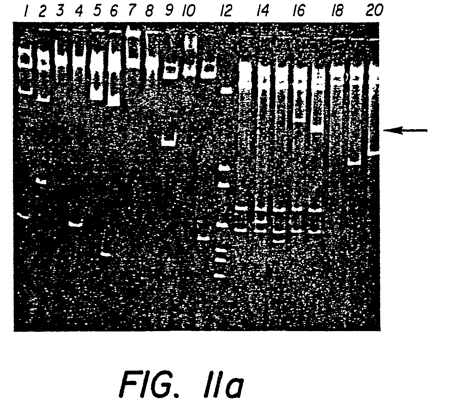

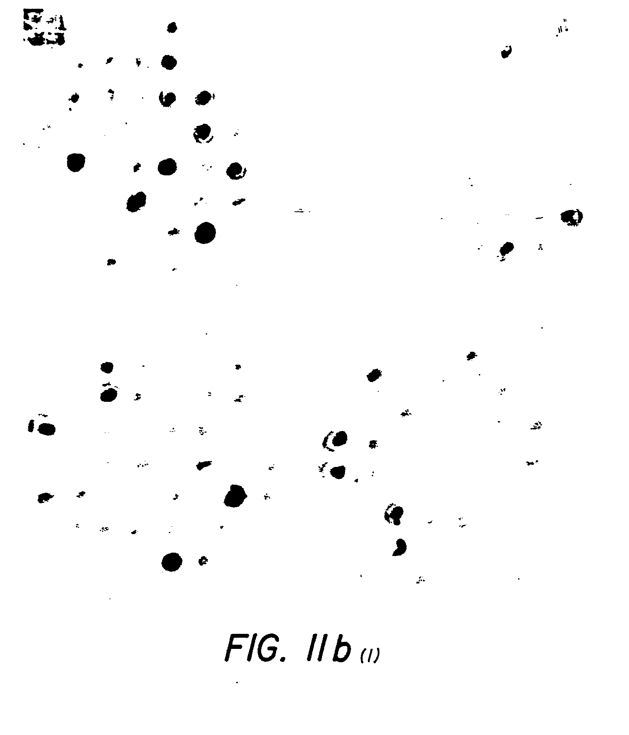

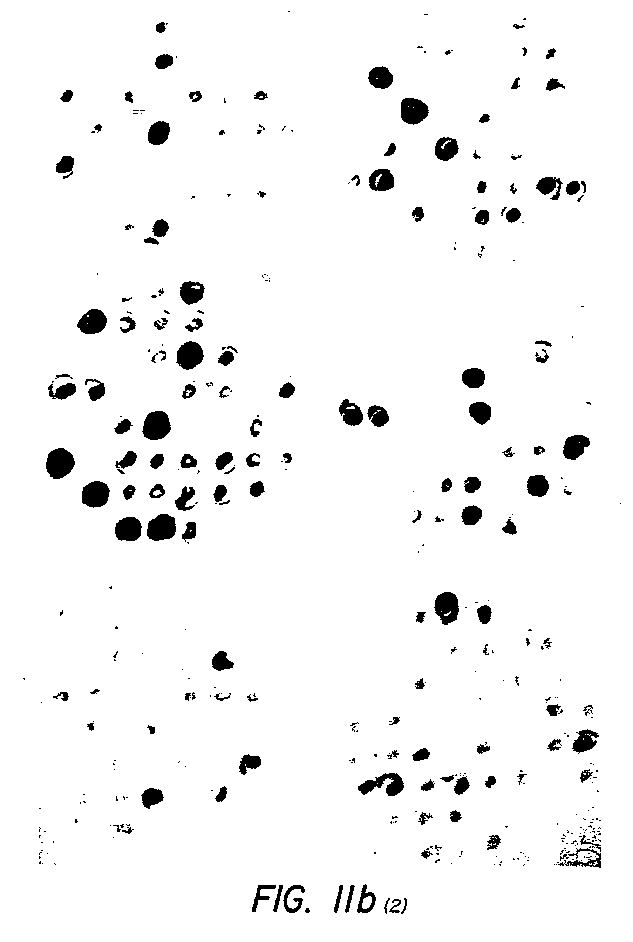

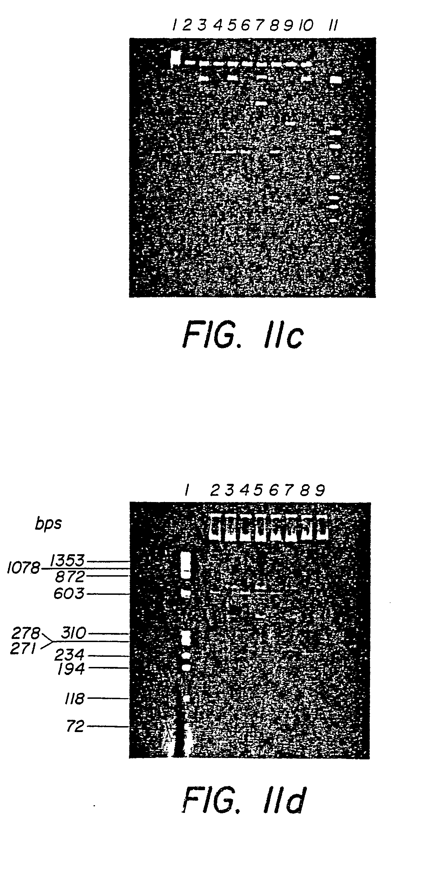

Fig. 11 refers to checking the results from the construction of PR84. Fig. 11a is a photograph referring to the restriction digestion of plasmids PR81, PR 8/ms/34, pSVod and 520.

Lane  1   PR81—*Hind*III/*Eco*RI
     2   PR81—*Xho*I/*Eco*RI
     3   PR81—*Nco*I
     4   PR81—*Nco*I/*Xho*I
     5   PR 8/ms/34—*Hind*III/*Eco*RI
     6   PR 8/ms/34—*Xho*I/*Eco*RI
     7   PR 8/ms/34—*Nco*I
     8   PR 8/ms/34—*Nco*I/*Xho*I
     9   pSVod—*Nco*I/*Sa*/I
   10   pSVod—*Pvu*II/*Sa*/I
   11   pSVod—*Hind*III/*Eco*RI
   12   pSVod—*Hin*fI STANDARD
   13   520—*Pvu*II
   14   520—*Pvu*II/*Xba*I
   15   520—*Pvu*II/*Xho*I
   16   520—*Pvu*II/*Eco*RI
   17   520—*Pvu*II/*Nco*I
   18   520—*Nco*I
   19   520—*Nco*I/*Xba*I
   20   520—*Nco*I/*Xho*I

Figs. 11b(1) and 11b(2) refer to a Grunstein colony hybridization analysis of PR 84-like plasmids. A nick translated radioactive probe (approximately $10^8$ cpm/μg) of the plasmid 51 Sau 3A/Xho I *Drosophila* control element fragment was used in these assays.

Fig. 11c refers to a 5% polyacrylamide gel analysis of the *Xho*I digestion products of PR84-like plasmids selected from the positive colonies identified in Fig. 11b.

| Lane | 1 | filter 3, Sample 12 | —*Xho*I |
|---|---|---|---|
| | 2 | filter 3, Sample 15 (PR82) | —*Xho*I |
| | 3 | filter 3, Sample 22 (PR82) | —*Xho*I |
| | 4 | filter 3, Sample 42 (PR84) | —*Xho*I |
| | 5 | filter 3, Sample 45 (PR84) | —*Xho*I |
| | 6 | filter 4, Sample 19 (PR84) | —*Xho*I |
| | 7 | filter 4, Sample 40 (PR84) | —*Xho*I |
| | 8 | filter 5, Sample 16 (PR84) | —*Xho*I |
| | 9 | filter 5, Sample 28 (PR84) | —*Xho*I |
| | 10 | filter 5, Sample 39 (PR84) | —*Xho*I |
| | 11 | pSVod (PR84) | —*Hin*fI STANDARD |

Fig. 11*d* refers to further restriction analysis of plasmids giving the *Xho*I digestion pattern shown in lanes 2, 4, 6 and 8 of Fig. 11*c* Analsysis was performed as described for Fig. 11*c*.

| Lane | 1 | Ø×174 RF—*Hae*III STANDARD |
|---|---|---|
| | 2 | filter 5, Sample 16—*Xba*I/*Nco*I |
| | 3 | filter 5, Sample 16—*Xho*I/*Nco*I |
| | 4 | filter 4, Sample 19—*Xba*I/*Nco*I |
| | 5 | filter 4, Sample 19—*Xho*I/*Nco*I |
| | 6 | filter 3, Sample 42—*Xho*I/*Nco*I |
| | 7 | filter 3, Sample 42—*Xho*I/*Nco*I |
| | 8 | filter 3, Sample 15—*Xba*I/*Nco*I |
| | 9 | filter 3, Sample 15—*Xho*I/*Nco*I |

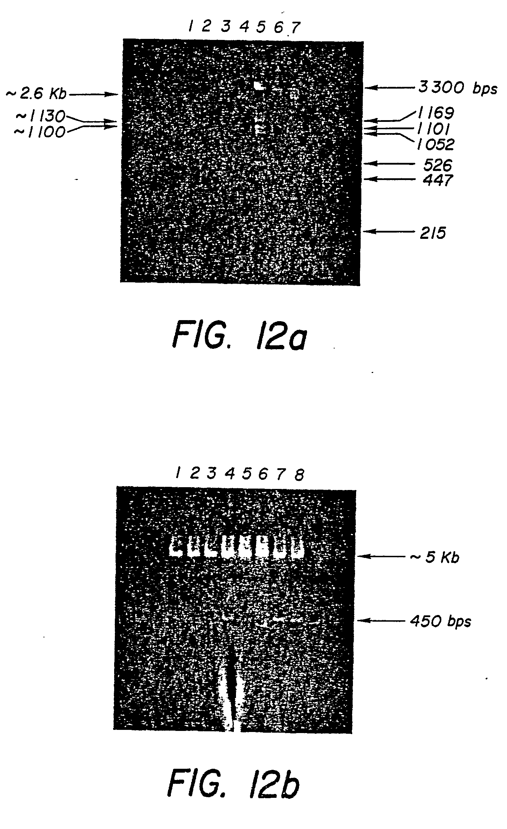

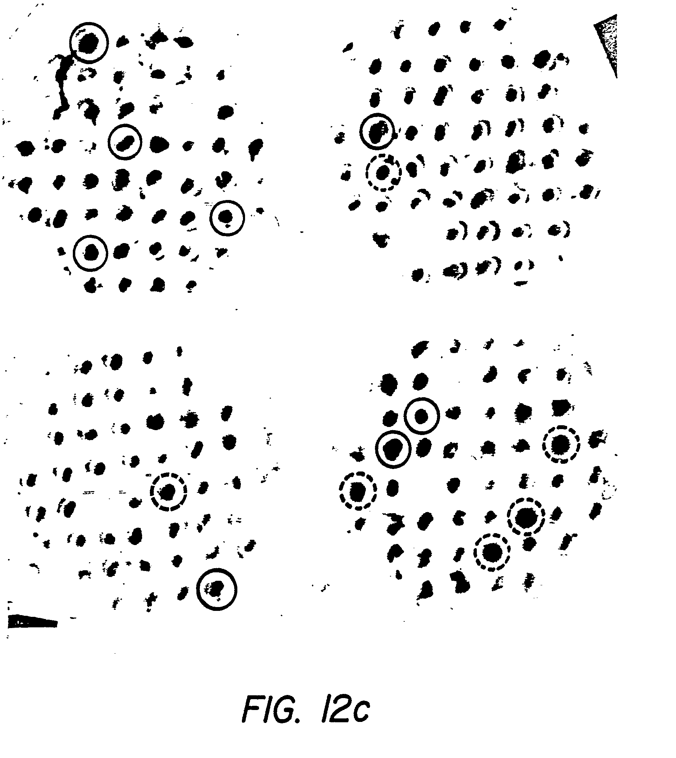

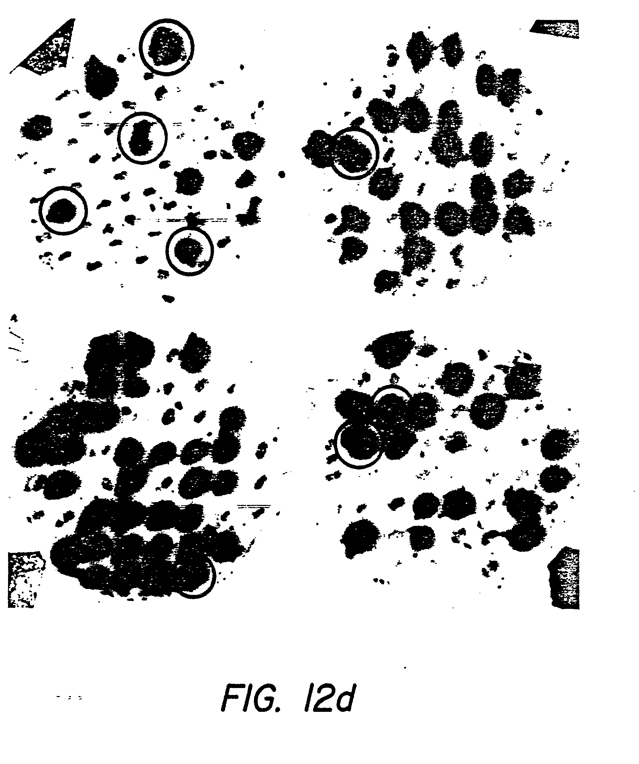

Fig. 12 describes the characterization of plasmid p629.
Fig. 12*a* refers to a 5% polyacrylamide gel analysis of some candidate 629 series plasmids.

Plasmid

| Lane | 1 | 629/8 | *Bg*III/*Eco*RI |
|---|---|---|---|
| | 2 | 629/7 | *Bg*III/*Eco*RI |
| | 3 | 629/6 | *Bg*III/*Eco*RI |
| | 4 | 629/4 | *Bg*III/*Eco*RI |
| | 5 | DNA standard | |

Fig. 12*b* refers to another 5% polyacrylamide gel analysis of all eight candidate 629 series plasmids.

Plasmid

| Lane | 1 | 629/1 | *Xho*I |
|---|---|---|---|
| | 2 | 629/2 | *Xho*I |
| | 3 | 629/3 | *Xho*I |
| | 4 | 629/4 | *Xho*I |
| | 5 | 629/5 | *Xho*I |
| | 6 | 629/6 | *Xho*I |
| | 7 | 629/7 | *Xho*I |
| | 8 | 629/8 | *Xho*I |

Fig. 12*c* shows a Grunstein colony hybridization assay using the nick translated *Xho*—*Bg*III fragment from plasmid p622b.
Fig. 12*d* shows a Grunstein colony hybridization assay using the nick translated *Hin*dIII/*Bam*HI · HA gene fragment from plasmid A/PR 8/mx/34.

Description of the specific embodiments

Methods and compositions are provided for the controlled expression of a gene in a eukaryotic host. The present invention is particularly useful for expression mammalian genes in mammalian hosts, typically in a host cell culture similar or identical to the host in which the gene is naturally expressed. In this way, post-transcriptional and post-translational modifications of the gene products will be achieved, thus assuring the competence of such resulting gene products.

DNA sequences which comprise an inducible expression control region derived from a eukaryotic heat-shock protein (hsp) gene are employed. Such control regions are inducible for example by elevated temperatures and include sequences responsible for both transcriptional and translational control of the hsp gene activity, e.g., the promoter region (RNA polymerase recognition and binding sites), and possibly also heat-shock protein gene control sequences which have been modified in such a way that the allow the constitutive expression of downstream coding sequences. The control region may also include other portions of the translated and untranslated regions of the hsp gene which participate in transcriptional and/or translational regulation including 3'untranslated sequences. Furthermore, sequences derived from the 3' untranslated region flanking the structural gene (i.e. the noncoding portion of the gene of interest) may be included. Conveniently, the entire hsp gene including the structural region as well as the 5' and 3' flanking regions may be employed. In the latter case, the gene of interest may be inserted internal to the structural region to produce a fused protein upon translation. The protein of interest may then be recovered by conventional means.

The term heat-shock protein (hsp) gene will be employed in the specification and the claims to denote the entire gene locus responsible for the inducible expression of heat-shock proteins, specifically including the 5' regulatory sequences upstream of the structural region, the structural region which encodes the mRNA transcript (including untranslated leader sequences) and the 3' flanking region downstream of the structural gene.

Heat-shock protein genes may be obtained from host higher eukaryotic organisms. Particular heat-shock proteins have been identified in fruit flies (*Drosophila*), mice, frogs, monkeys, and man. Heat-shock genes suitable as a source for the control regions of the present invention may be obtained from any of these or other eukaryotic organisms. Although heat-shock genes derived from one species can be expressed in other species and even in procaryotes, it is generally desirable that in order to optimize the expression advantages of heat-shock control elements at both the transcriptional and the translational levels, these elements should be derived from the same or similar organism or cell type that will be used for expression of the gene of commercial interest. For instance, constructions including genes under control of *Drosophila* heat-shock elements can be most advantageously expressed in *Drosophila* cells in culture.

In the Experimental section hereinafter, an inducible expression control region obtained from a heat-shock protein gene which encodes for a 70 kdal heat-shock protein found in *Drosophila* was used to express *E. coli* β-galactosidase gene in *E. coli*, COS I Monkey cells, and in *Xnopus* occytes. The same region was used to control the expression of the influenza haemagglutinin gene in COS I cells.

The inducible expression control region of the present invention may be combined with an extrachromosomal replication system for a predetermined host to provide an expression vector for that host. Such vectors will include DNA sequences having restriction site(s) for insertion of gene(s) 3' to said control regions to provide the regulated transcription and translation of the inserted genes. The vector can also include markers for selection in bacteria and in eukaryotic host cells, a prokaryotic replication system allowing cloning of the vector in a prokaryotic host, and other DNA regions of interest.

Alternatively, the expression control region can be joined to a desired structural gene and the resulting DNA constructs introduced directly into the host cells. Methods for such direct transfer include injection of the DNA into nuclei (Cappechi (1980) cell *22:* 479—488) and co-transformation by calcium phosphate precipitation (Wigler *et al.* (1979) Cell *16:* 777—785) or DEAE dextran (McCutchan, J. H. and Pagano J. S. (1968) J. Nat. Cancer Inst. *41:* 351—357).

As stated above, the DNA constructs of the present invention may include differing portions of the hsp gene. The constructs will include at least the 5' regulatory region which carries the promoter, regulatory sequences such as operators, activators, cap signals, signals enhancing ribosomal binding, and other sequences as well as additional DNA leader sequences responsible for transcriptional and translation control. The DNA constructs may also include part of the protein coding sequence of the hsp gene, resulting in the production of fused proteins when the foreign gene to be expressed is inserted downstream of the hsp sequences. If a fused protein is not desired, it will be necessary to remove the hsp coding sequences from the isolated hsp DNA by usual methods such as restriction by enzymes and exonuclease digestion.

It may be desirable to leave at least a portion of the hsp structural gene downstream from the natural translational initiation codon. In this way, a fused protein including the amino-terminal amino acid sequences of the heat-shock protein is provided. When such fused proteins are produced, it may be desirable to introduce selective cleavage sites so that the desired protein can be separated from the precursor protein. See U.S. Patent No. 4,366,246 to Riggs which teaches how such cleavage sites may be introduced.

The expression control region of the present invention will usually be combined with a terminator for complete transcriptional control of the inserted structural gene. Conveniently, the terminator can be derived from the heat-stock gene itself, although the inserted structural gene may carry its own or any other suitable terminator sequence.

Extrachromosomal replication system may also be used. Suitable replication systems include autonomously replicating sequences as described by Struhl *et al.* (1979) PNAS *73:* 1471—1475 and the 2 μm plasmid for replication in yeast. Mammalian replication systems could be derived from papovaviruses, such as simian virus 40 and bovine papilloma virus; adenoviruses; avian retroviruses, such as avian sarcoma virus; and mammalian retroviruses such as Moloney leukemia virus.

In addition to the optional eukaryotic replication system, it is advantageous to provide a prokaryotic replication system to allow for cloning of the vector in a bacterial host. This allows large quantities of the vector to be grown in well characterized bacterial systems prior to transforming a eukaryotic host. Suitable prokaryotic replication systems are well known and include plasmids such as pBR322, pRK290, ColE1, and bacteriophages, e.g. λdv. The prokaryotic replication systems will necessarily include an origin of replication recognizable by a prokaryotic host, and will usually include one or more markers for the selection of transformants in the prokaryotic host. Such markers include biocide resistance, toxin resistance, and the like. Alternatively, complementation allowing the growth of an auxotrophic host in a selective medium may be employed. Such techniques are well known in the art and need not be described further.

Usually, the markers employed will be different for selection in prokaryotic and eukaryotic hosts. Various dominantly acting markers are useful in selecting for transformed mammalian cell lines. They usually comprise a specific gene whose expression confers a new drug-resistant phenotype to the mammalian cells in an appropriate selective medium. Specific markers include the bacterial xanthine-guanine phoshoribosyl transferase gene which can be selected in medium containing mycophenolic acid and xanthine (Mulligan et al. (1981) PNAS 78: 2072—2076); transformants with vectors carrying a mouse cDNA fragment coding for dihydrofolate reductase may be selected for using medium containing aminopterin (Subramani et al. (1981) Mol. Cell Biol. 1: 854—861); and a bacterial plasmid gene specifying an amino-glycoside phosphotransferase that inactivates the anti-bacterial action of neomycin-kanamycin derivatives may be selected for using medium containing G418 a neomycin derivative toxic for most mammalian cell lines (Colbere-Garapin et al. (1981) J. Mol. Biol. 150: 1—14).

It is evident that the number of copies of gene expression units introduced into a host cell may vary and that by proximal combination of one of the amplifiable genes such as the mouse dihydrofolate reductase gene the number of copies of integrated gene expression units may likewise be amplified by induction of the amplification of the associated amplifiable gene and the proximal DNA. See for example the co-amplification of dihydrofolate reductase cDNA and the E. coli XGPRT (xanthine-guanine phorphoribosyl transferase) gene in Chinese ovary cells (Rungold, G. et al., (1981). J. Mol. and Appl. Genetics, 1, 165—175).

In the exemplary method for preparing the vectors of the subject invention, both the expression control region of the heat-shock protein gene and the eukaryotic replication system are inserted into a suitable prokaryotic plasmid. The manner and order of the insertion are not critical, and it is necessary only that the resulting vector retains viable replication systems for prokaryotic and when necessary eukaryotic hosts.

The DNA constructs containing Drosophila heat-shock gene control segments functionally linked to a structural gene of interest can be used to synthesize products of said gene in Drosophila cells or cultured cells closely related to Drosophila. Since the structural gene is under the transcription and if necessary translation control of the heat-shock control element, its expression can be enhanced by for example increasing the ambient temperature of the cells for instance in the range 37—42°C. By such induction a large fraction of the newly made mRNA will be derived from said structural gene. Now if the structural gene is a human gene encoding a protein requiring complex processing, the expression vector preferably comprises, in addition to said structural gene, replication elements and marker genes, a heat-shock control element derived from a human heat-shock gene. This construct will be introduced advantageously into cultured human cells by procedures described above to produce products of said human gene. As necessary the choice of recipient human cells will be made on the basis of their competence in correctly expressing the fully processed gene product.

A wide variety of structural genes may be introduced into the subject vectors to permit the production of various gene products including polypeptides, such as enzymes, proteins, hormones, novel protein structures and the like.

Experimental

The following examples are offered by way of illustration.

1. The construction of plasmid pRV15 which contains the E. coli β-galactosidase gene under the control of a Drosophila heat-shock control element.

A 650 base pair (bp) DNA fragment from one of the two Drosophila 70 kdal heat-shock protein genes, containing a heat-shock gene transcription control element, a complete RNA leader sequence, a translation initiation signal and a sequence coding for the first few amino acids of the Drosophila 70 kdal heat-shock protein, was obtained from a sub-clone of part of plasmid 132E3 (Schedl et al. (1978) Cell 14: 921—929; see Fig. 1). Plasmid 132E3 contains two complete genes for the 70 kdal heat-shock protein, and the aforementioned isolated sub-clone, plasmid 51 (Karch et al. (1981) J. Mol Biol. 148: 219—230), contains a fragment of the first heat-shock gene in plasmid 132E3. This fragment was isolated by digestion of plasmid p51 with the restriction endonucleases Bg/II and BamHI.

The upper part of Fig. 1a is a representation of a portion of plasmid 132E3 indicating the two genes encoding 70 kdal heat-shock protein (thick line segments) and some of the identified restriction sites. The lower part of Fig. 1a represents a portion of p51 containing the Bg/II—BamHI segment containing the aforesaid 650 bp fragment, bounded by the Sau3A cleavage sites.

Fig. 1b is a more detailed representation of the portion of interest contained in p51 with additional restriction sites indicated, and also showing the position of the 650 bp fragment between two Sau3A sites.

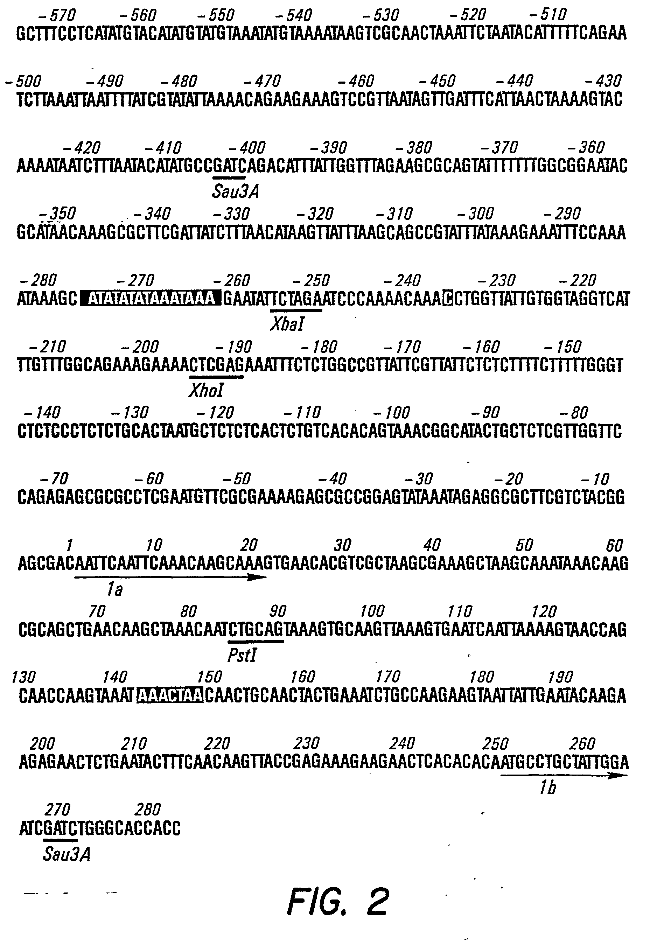

Fig. 2 provides the DNA sequence data for one strand contained in the aforementioned 650 base pair fragment. The limits of this sequence are indicated by the position of the Sau3A restriction sites (GATC). Also, XbaI, XhoI and PstI recognition sites for restriction are indicated, and the position of the transcription and translation start sequence are indicated by arrows 1a and 1b, respectively. The 650 bp fragment was functionally inserted into plasmid pMC1403 in a position to control the expression of test genes in this plasmid. Plasmid pMC1403 (Casadaban et al. (1980) J. Bacteriol. 143: 971—980) is a derivative of plasmid pBR322 (Bolivar et al. (1977) Gene 2: 95—113) containing the entire E. coli lac operon with the exception of the sequences coding for the first seven amino acids of β-galactosidase and all sequences 5' to the β-galactosidase protein coding region (promoter, ribosomal binding site, translation initiation codon). Consequently lac strains of E. coli such as MC 1061 (Casadaban et al. (1980) J. Mol. Biol. 138: 179—207) carrying plasmid pMC1403 do not produce β-galactosidase. A polylinker (EcoRI, SmaI, BamHI) at the 5' end of the lac sequences in pMC1403 permits the introduction of foreign DNA sequences upstream from the β-galactosidase coding region. Insertion of a segment containing a functional promoter and the RNA leader sequences will result in the production of β-galactosidase activity. It should be noted that the amino-terminal end of β-galactosidase is not essential for its enzymatic activity (Muller-Hill B. and Kania, J. (1974) Nature 249: 561—563). All that is therefore required is that the inserted promoter/RNA leader sequence permits reading in the correct frame with the incomplete β-galactosidase coding region. Hence the 650 bp segment was ligated into the BamHI site of plasmid pMC1403, in front of the incomplete β-galactosidase gene. The new recombinant plasmid thus obtained (see Fig. 3) was designated pRV15. The procedure of this plasmid construction is shown in a diagrammatic form in Fig. 3. In this figure, the upper left part (Fig. 3a) represents a section of the aforementioned plasmid 51 containing the 650 bp fragment 2 to be cleaved out with Sau3A and to be inserted into the BamHI site of plasmid pMC1403 shown on Fig. 3b. The truncated lac operon of the plasmid pMC1403 is represented by the heavy line with numeral 3 on the diagram of Fig. 3b. It also comprises a structural gene for ampicillin resistance. Plasmid p51 is digested with Sau3A to excise the aforesaid 650 bp fragment represented on the left of Fig. 3c by numeral 2; this fragment was purified from polyacrylamide gels (see Fig. 5c) by electroelution. This fragment was inserted into the BamHI site of pMC1403 in either orientation. The ligation mixture was then used to transform the lac strain of E. coli MC 1061. Transformants were plated on media containing ampicillin and Xgal (Xgal is 5 - bromo - 4 - chloro - 3 - indolyl - N - D - galactoside) which is a substrate for β-galactosidase, and which after cleavage by the enzyme produces an identifiable colored product (see Miller, J. Experiments in Molecular Genetics, pp. 47—55, Cold Spring Harbor, N. Y. 1972). Plasmid pMC1403 did not produce β - galactosidase activity in this assay while plasmid pRV15 and a large number of other transformants, prepared in the aforesaid manner, were found to produce substantial amounts of β - galactosidase as determined by the color change produced on Xgal plates.

In addition, β-galactosidase-coding plasmid constructions such as pRV15 were analyzed further by the colony hybridization assay of Grunstein (Grunstein and Hogness (1975) PNAS 72: 3961—3966) using either radioactively labelled 650 bp Sau3A fragments from p51 (see Fig. 4b) or the 2 kbp XbaI gene fragment (see Figs. 1a, 4a) from plasmid 132E3 as hybridization probes. These fragments were radioactively labelled by the process of nick translation (Maniatis et al. (1975) PNAS 72: 1184). As seen in Figs. 4a and 4b, all selected transformants hybridized to both radioactive probe DNAs demonstrating the presence of both DNA sequences in recombinant plasmids such as pRV15.

The presence of the 650 bp fragment containing the Drosophila 70 kdal heat-shock gene control element was confirmed by restriction analysis, see Figs. 5a, b and c. Plasmid pMC1403 contains unique restriction sites for EcoRI and SalI, but no XhoI or XbaI sites. The Sau3A 650 bp fragment from plasmid p51 however contains unique sites for restriction by XhoI and XbaI but has no sites for restriction enzymes such as EcoRI and SalI. In contrast, recombinant plasmids such as pRV15 should contain unique sites for all four aforementioned enzymes (see Fig. 3). Restriction of recombinant DNA from plasmids such as pRV15 with XhoI and SalI or with XbaI and SalI should produce two fragments of about 4 and 6.5—7 kbp if the structure shown for pRV15 in Fig. 3 is correct. Plasmid pMC1403 however should only be linearized. Fig. 5a shows a photograph of DNA fragments produced by the aforementioned restriction enzymes after electrophoresis on agarose gels, and confirms the structure of plasmid pRV15 shown in Fig. 3. These results are presented again in Fig. 5b where it is also demonstrated that digestion with SalI, XhoI or XbaI individually only linearize pRV15, further confirming the structure shown in Fig. 3.

Further, if pRV15 contains the 650 bp Sau3A fragment of plasmid p51 inserted into the BamHI site of pMC1403, then it should be possible to recover the 650 bp Sau3A fragment by digestion of recombinant DNAs such as pRV15 with the restriction enzyme Sau3A. That this is the case is shown in Fig. 5c lane 7 by the arrows. In addition, the identity of the 650 bp excised fragment is confirmed by its restriction by XbaI or by XhoI. See Fig. 5c lanes 5 and 6.

The demonstration that the Sau3A 650 bp fragment and the β-galactosidase coding sequences are in the orientation shown for pRV15 in Fig. 3 is presented in Figs. 6a and b. In Fig. 6a, the correct orientation is shown in a diagrammatic form from which the respective double digestions with EcoRI and XbaI or by EcoRI and XhoI would predict the excision of fragments from the 650 bp fragment, correctly oriented with respect to the β-galactosidase gene 12, of respectively 150 and 220 bp. In Fig. 6a, the arrow 11 indicates the transcriptional direction of the heat-shock control elements. The above prediction is demonstrated by the restriction gel analysis shown in Fig. 6b where EcoRI and XbaI double-digestion liberate an approximately 150 bp fragment, and EcoRI, XhoI double-digestion, a 220 bp fragment as evidenced by electropheresis on 5% acrylamide gels. These experiments confirm that plasmid pRV15 has the desired orientation of control and coding elements shown in Fig. 3. The position of bands of sizes 515, 220 and 154 bp are indicated by arrows marked with the corresponding numbering.

The hybrid plasmid pRV15 and other identical isolates constructed as described in the preceding section, produce substantial amounts of β-galactosidase as determined by examining the color changes on Xgal plates. The presence of β-galactosidase protein produced in *E. coli* under the control of a *Drosophila* heat-shock control element has also been clearly demonstrated by immune precipitation of protein extracts of *E. coli* containing plasmid pRV15 after radioactive labelling a newly synthesized protein with $^{35}$S-methionine. A polypeptide of molecular weight approximately 120,000 daltons was precipitated from such protein extracts by immune sera directed against authentic *E. coli* β-galactosidase.

The details of the construction and expression of pRV15 are now set forth.

A. Construction of plasmid pRV15

10 μg of plasmid p51 (Fig. 1a, lower part) were digested for 4 hours at 37°C with 10 units of *Sau*3A (incubation buffers for this, and all other digestion described below as suggested by the supplier of the restriction enzymes: New England Biolabs Cat. 1982). The concentration of DNA during digestion with *Sau*3A was 40 μg/ml. The digestion products were electrophoresed on a non-denaturing 5% polyacrylamide gel in 1×TBE(10.9 g Tris base, 5.5 g boric acid, 0.93 g Na$_2$EDTA per liter H$_2$O) buffer. A *Sau*3A digest of pBR322 was electrophoresed in parallel and served to identify the 650 bp 51 *Sau*3A promoter fragment (Fig. 5c). DNA fragments were visualized with ethidium bromide (EtBr). The region containing the promoter fragment, indicated by the arrows in Fig. 5c, was cut out of the gel and the fragment was electroeluted into a dialysis bag. Electroelution was carried out for 5 hours at 200v in a 1×TBE buffer. The eluate was collected in a 15 ml siliconized Corex tube and ethanol-precipitated overnight at −20°C. The precipitate was collected by centrifugation (Sorvall, 30 min., 10,000 rpm, SS34 rotor), dried in a lyophilizer and resuspended in 200 μl of TE buffer (10 mM Tris. HCl, pH 7.5, 1 mM Na$_2$EDTA). The DNA was then extracted twice with TE-saturated phenol and twice with ether. The solution was then passed through a Sephadex G75 mini-column (in a Pasteur pipet). DNA in the column eluate was ethanol-precipitated twice, dried and resuspended in 20 μl of TE buffer. A 10 μl portion was incubated with several units of *Xba*I in the appropriate digestion buffer for 1 hour at 37°C. The partially digested DNA was electrophoresed on a 5% polyacrylamide gel. This experiment (Fig. 5c) demonstrated that the isolated segment was the 650 bp *Sau*3A fragment, since the promoter fragment includes the only *Xba*I site of plasmid p51.

One μg of plasmid pMC1403 was digested with 4 units of *Bam*HI for 30 min. at 37°C. The digested DNA was extracted 3 times with phenol and then 3 times with ether. The DNA was further purified by 2 subsequent ethanol precipitations. The pelleted DNA was dried and then resuspended in 20 μl of TE buffer.

Ten μl aliquots of the solutions containing the 650 bp promoter fragment and digested pMC1403 were combined and then incubated overnight at 14°C with an excess of T4 DNA ligase in a total volume of 25 μl (incubation buffer was recommended by New England Biolabs 1982 Cat.). The ligation mixture was then incubated for several hours with 8 units of *Bam*HI at 14°C. Aliquots (1, 3 and 7 μl) were then used to transform 0.5 ml aliquots of CaCl$_2$-treated *E. coli* MC 1061. Aliquots (0.1 ml) of the transformation suspension were then placed on LB agar containing 10 μg/ml ampicillin and 40 μg/ml of Xgal. Blue colonies were observed after overnight incubation of the plates at 37°C. Approximately 80 β-galactosidase-producing colonies were isolated. The presence of the p51 *Sau*3A promoter fragment in the recombinants was established by Grunstein colony hybridization (Fig. 4b). The hybridization probes were prepared as follows: the p51 *Sau*3A promoter fragment was isolated as described above. 50 μg of 132E3 were digested with 50 units of *Xba*I for 2 hours at 37°C. The digestion products were separated on a 0.9% agarose gel. The 2 kbp 70 kdal heat-shock protein gene fragment was eluted electrophoretically from the gel and was purified as described above. The latter fragment and the p51 *Sau*3A fragment were then $^{32}$P-labelled by nick translation to a specific radioactivity of $2 \times 10^8$ cpm/μg. The two probes were then denatured and hybridized to two nitrocellulose filters containing DNA of the 80 selected transformants and of pMC1403 (Figs. 4a, b). Both probes strongly hybridized to DNAs of all 80 recombinants, suggesting that all recombinants contained the 650 bp promoter fragment. Clones 5, 15, 25, 35, 45 and 55 were selected for further studies. Small quantities of DNA were prepared from each of these clones (Davis *et al.* (1980) Methods in Enzymology, Grossmann and Moldave, eds. *65:* 404—414). These DNAs and pMC1403 DNA were compared further by restriction digestion and electrophoresis on 0.9% agarose gels (Figs. 5a, b). The results indicate that restriction of the recombinant plasmids such as pRV15 with *Xho*I and *Sal*I or with *Xba*I and *Sal*I produce two fragments of 4 and 6.5—7 kbp, thus confirming the structure of plasmid pRV15 as shown in Fig. 3d.

B. Expression of the *Drosophila* heat-shock—*E. coli* β-galactosidase fusion gene in *E. coli*

Bacteria containing the hybrid plasmid pRV15 and other isolates constructed as described in the preceding section, produce substantial amounts of β-galactosidase as determined by examining the color changes on Xgal plates. The presence of a β-galactosidase protein produced in *E. coli* under the control of a *Drosophila* heat-shock control element was also demonstrated by immune precipitation (Bromley *et al.* (1979) J. Virol. *31*: 86—93) of protein extracts of *E. coli* containing plasmid pRV15 after radioactive labelling of newly synthesized proteins with $^{35}$S-methionine. A polypeptide of molecular weight about 120,000 daltons was clearly precipitated from such protein extracts by immune sera directed against authentic *E. coli* β-galactosidase.

C. Expression of a heat-shock β-galactosidase hybrid gene in Xenopus oocytes

Plasmid pRV15 (50—100 µg) was digested with an excess of *Sal*I and *Eco*RI. The two resulting restriction fragments were then separated on 0.85% agarose gels. The 7kb fragment containing the hybrid gene but no vector sequences was purified by electroelution and gel filtration on Sephadex® G75. This fragment was then incubated with an excess of T4 DNA ligase in a total volume of 25 µl as described above, to permit circle formation. The ligated fragments were injected into oocytes as described by Voellmy and Rungger (1982) PNAS *79:* 1776—1780. Following a 6—20 hours preincubation at 20°C, the oocytes were injected a second time of α-$^{32}$P-gtp and either heat-treated for 2 hours at 37°C or kept at 20°C for the same period. Total RNA prepared from these oocytes or from oocytes that did not contain foreign DNA was hybridized to Southern blots of Sal I/Eco RI digests of plasmid pMC1403. Oocytes that did not contain the hybrid gene did not produce measurable amounts of RNA complementary to the β-galactosidase gene. Both heat-treated and untreated oocytes containing the pRV15 fragment were found to have made β-galactosidase RNA in approximately similar quantities.

In another series of experiments, unlabelled RNA was isolated from oocytes containing the hybrid gene which had been either heat-treated for 2 hours or incubated at 20°C for the same time in the presence or absence of low concentration of α-Amanitin (Voellmy and Rungger (1982) PNAS *79:* 1776—1780). The oocyte RNAs were labelled by reverse transcription as described by Bromley *et al.* (1979) J. Virol. *31:* 86—93 and hybridized to pMC1403 Southern blots. Again β-galactosidase transcripts were present in RNAs for heat-treated and untreated oocytes. α-Amanitin did stop transcription of the hybrid gene at both temperatures indicating that RNA polymerase B (i.e. II) was responsible for all observed transcriptional activity.

2. The construction and use in eukaryotic cells of plasmid PR84 which contains a human influenza virus haemagglutinin gene under the control of a *Drosophila* heat-shock control element

A plasmid denoted 520 was constructed from plasmid pRV15 and plasmid pSVod (Mellon *et al.* (1981) Cell *27:* 279—288) which is capable of replicating either in procaryotic cells or in certain eukaryotic cells. Plasmid 520 allows rapid analysis of transcriptional control since it includes the SV40 virus origin of replication and is able to replicate efficiently in SV40 mutant-transformed, transformation antigen positive COS cells (Gluzman (1981) Cell *23:* 175—182).

The schematic form of the construction of plasmid 520 is shown in Fig. 7, Plasmid pRV15 (Fig. 7*a*, representation in linear form) was digested with *Sma*I, subsequently digested with *Sal*I and the resulting 7 kbp fragment (Fig. 7*c*) containing the 650 bp *Drosophila* control element 2 and the lac gene fragment 3 originating from pMC1403 present in pRV15 was isolated by electrophoretic elution from a preparative agarose gel.

Plasmid pSVod (Fig. 7*c*) was digested with *Bam*HI, the cohesive ends were filled in with DNA polymerase Klenow fragment and the DNA finally digested with *Sal*I. In Fig. 7*c*, numeral 10 indicates the site of the deletion of the 1kb pBR322 sequence which has been claimed to be inhibitory for replication in eukaryotic cells. The truncated pSVod fragment (Fig. 7*d*) so formed was ligated to the 7 kbp fragment isolated from pRV15, and the ligation mixture was used to transform *E. coli* MC1061. The structure of plasmid 520 is shown in Fig. 7*e* in which a more detailed representation of the 650 bp segment also appears with the *Xba*I, *Xho*I and *Pst*I restriction sites. Another restriction site (*Pvu*II) is also indicated and corresponds to the CAGCTG sequence between 60 and 70 bp, as denoted in Fig. 2. The significance of the *Pvu*II site will be mentioned hereinafter. Plasmid 520 is shown in circularized form in Fig. 8*a* where the segments a, y and z represent genes of the lac operon. Numeral 11 designates the fragment containing the SV40 origin.

Transformants were plated out on Xgal-Ampicillin plates as described previously, and blue transformants suspected of containing plasmid 520 were isolated. From the structure shown in Fig. 8*a*, plasmid 520 should provide the following size fragments were digested with the named restriction enzyme combinations.

| Fragment sized in kbp | Enzymes |
|---|---|
| 0.75/1.1/8.5 | *Pst*I/*Eco*RI |
| 2.6/7.2 | *Hind*III/*Sal*I |
| 3.1/6.7 | *Sal*I/*Xba*I |

The 520 plasmids indeed produced fragments of the correct sizes when digested as evidenced in the gel analyses of Figs. 8*b, c* and *d*. Other fragments produced by various combinations of enzyme digestions serve as controls for the interpretion of the digestions noted above.

Plasmid p81 places a gene which encodes for a eukaryotic protein in the correct reading frame with a 650 bp heat-shock control element of p520. As starting material for this construction, plasmid A/PR 8/ms/34 (Mount Sinai) Clone No. 4.76 containing a human influenza haemagglutinin gene (1775 bp, numeral 20)

inserted into the *Pvu*II site of vector PAT 153/Pvu II/8 was obtained from Dr. G. Brownlee (University of Oxford, Department of Pathology U.K.). The plasmid containing the haemagglutinin (HA) gene (insert 20, see Fig. 9) was digested with *Bam*HI, and the ends were filled in with DNA polymerase Klenow fragment. Following digestion with *Hin*dIII, the haemagglutinin gene fragment was purified on agarose gels. The insert 20 contains a complete HA protein coding sequence, an RNA leader segment, and 40 bp of 3' nontranslated sequence. The HA gene-containing fragment was inserted into plasmid vector pSVod to produce plasmid PR81 as follows: pSVod DNA was digested with *Sal*I, ends were filled in as described above, and the DNA was further digested with *Hin*dIII (see structure in Fig. 9*b*). The HA fragment 20 and the *Sal*I/*Hin*dIII digest of pSVod were ligated together using T4 DNA ligase in the presence of *Bam*HI and *Sal*I to give PR81 (see Fig. 9*c*), and the ligation mixture was used to transform *E. coli* C 600.

Transformants were analyzed for the presence of PR81-like plasmids (for predicted structure, see Fig. 9*c*) as follows: Grunstein colony hybridization was performed in a manner similar to that described earlier in connection with pRV15 (see Fig. 4). A nick translated probe of the HA gene (*Bam*HI/*Hin*dIII fragment from pA/PR 8/34/ms/4.76 kb) was prepared and hybridized to the PR81-like transformants (see Figs. 9*d* and 9*e*). Figs. 9*d* and 9*e* show the autoradiograms of eight colonies. It can be seen from Figs. 9*d* and 9*e* that this DNA probe hybridizes to most of the transformants. Negative controls are provided by the pSVod containing colonies indicated in the figure by arrows.

PR81-like plasmids were further characterized by restriction analysis, as shown in Fig. 9*f*. The structure indicated for PR81 in Fig. 9*c* is verified by the formation of DNA fragments of the following sizes after digestion with the restriction enzymes indicated below:

| Plasmids | Sizes of fragments kbp | Enzymes used |
|---|---|---|
| PR81 | 3.2, 1.5—1.6 | *Eco*RI |
| PR 8/34 | 3.8, 1.3 | *Eco*RI |
| pSVod | 3.3 | *Eco*RI |
| PR81 | 4.5 | *Hin*dIII/*Sal*I |
| PR 8/34 | 3.3, 1.7—1.8 | *Hin*dIII/*Sal*I |
| pSVod | 2.7, 0.6—0.7 | *Hin*dIII/*Sal*I |

The results provided in Fig. 9*f* indicate the presence of the above fragments.

The construction scheme for plasmid PR84 is presented in Figs. 10*a* to 10*e*. Briefly, plasmid PR84 places the HA gene under the control of expression control region of the *Drosophila* heat-shock gene described heretofore. The hybrid gene itself is placed under the replication control of plasmid pSVod. Digestion of plasmid 520 (Fig. 10*a*) with *Pvu*II and *Nco*I results in the formation of two fragments of about 1 kbp in length, one of which (Fig. 10*b*) contains part of the SV40 origin of replication sequence and part of the 650 bp heat-shock control element, including 400 bp of 5'-nontranscribed sequence and 60 bp of the RNA leader sequence. The two 1 kbp fragments were isolated by electrophoresis on preparative gels of 1.2% low melting agarose.

Plasmid PR81 (Fig. 10*c*) was digested with *Hin*dIII, the ends were filled up with DNA polymerase Klenow fragment, and the DNA was further digested with *Nco*I which has a unique cutting site in this plasmid situated within the SV40 origin of replication sequence (see Fig. 10*d*). The resulting PR81 fragments were ligated with the 1 kbp fragments isolated from plasmid 520 and the ligation mixture was used to transform *E. coli* C 600. The resulting transformants include plasmids such as PR84 whose predicted structure is shown in Fig. 10*e*.

A more detailed structure for PR84 including partial DNA sequence predictions are presented in Fig. 10*f* in the form of a single strand representation of plasmid sequences. As shown in Fig. 10*f*, PR84 comprises from the 5' end, successively, a 250 bp origin of replication segment of SV40 origin, a 346 bp sequence from pBR322, a 400 bp *Drosophila* 5' nontranscribed sequence (p51; p132E3, see Karch *et al.* (1981) J. Mol. Biol, *148*: 219—230) originally included in the 650 bp heat-shock control sequence, a *Drosophila* 65 bp hsp gene RNA leader fragment (base pairs numbered 1 to 65), a linker segment of 8 base-pairs, a haemagglutinin (HA) RNA leader segment (base pairs numbered 1—32), a HA signal peptide coding segment (base pairs numbered 33—83) and a HA protein coding segment (bp 84 onwards).

The rather complicated construction scheme chosen for plasmid PR84 was checked by restriction digest analysis of the parent plasmids used, the results of which are shown in Fig. 11*a*. The following restriction sites are predicted for the plasmids:

PR81: 1 *Nco*I site, 1 *Hin*dIII site, the *Nco*I site in the SV 40 origin of replication sequence.

PR 8/ms/34: 1 *Nco*I site, no site in HA gene.

520: 1 *Nco*I site, several *Pvu*II sites in the lac operon a pair of 1 kbp *Nco*I/*Pvu*II fragments, one of them containing the origin—hsp 70 promoter fragment.

That these predictions fit with the structures of these plasmids illustrated in the construction schemes is shown by the results of the restriction analyses indicated by the gel patterns in Fig. 11*a*. The two 1 kbp *Nco*I/*Pvu*II fragments are clearly seen and are indicated in Fig. 11 (lane 17) by an arrow.

Further characterization of PR84-like plasmids was performed using the Grunstein colony hybridization procedure described before (see Fig. 4). In this case, a nick translated probe (approximately 10[8] cpm/µg) of

the *Sau*3A/*Xho*I fragment from plasmid p51 (containing the *Drosophila* hsp control element) was employed, and the results are presented in Fig 11*b*).

A number of the positive colonies identified in this way were grown up for the preparation of small amounts of each plasmid as described previously, and a series of restriction analyses were performed. The gel patterns so obtained are shown in Figs. 11*c* and *d*. The expected pattern of digestion products formed by *Xho*I digestion as predicted from the structure of PR84 presented in Fig. 10 are: one *Xho*I fragment of 405 bps and one very large fragment. Fig. 11*c* shows the results of this analysis and the plasmid clones in lanes 2, 4, 6 and 8 have the expected pattern as compared to the *Hin*fI standard digest pattern of pSVod. These plasmids were selected for further analysis where they were digested either with *Xho*I/*Nco*I or with *Xba*I/*Nco*I. The results obtained are shown in Fig. 11*d*. The expected sizes of fragments produced from the map shown in Fig. 10 would be one fragment of about 700 bp and one of 405 bp with *Xho*I/*Nco*I and one large fragment, and with *Xba*I/*Nco*I, one frgment of 650 bp and one large fragment. Fig. 11*d* shows the validity of these predictions for all of the plasmids analyzed.

DNA from plasmid PR84 was used to transfect eukaryotic cells. As suitable cells for bringing about such experiments COS cells (Gluzman (1981) Cell *23*: 175—182) were selected because of their usefulness for the rapid expression analysis of gene constructions (Gething and Sambrook (1981) Nature *293*: 620—625). After transfection, the cells were incubated at either 37°C or 42°C in the presence of labelled methionine ($^{35}$S). Then the contents of the cells were analyzed by immune precipitation using rabbit antiserum raised against PR 8/ms influenza virus (a gift of Dr. J. Skehel, M. R. C. Laboratories, Mill Hill, London U.K.). The complexes purified on protein A-Sepharose® were finally subjected to electrophoresis on polyacrylamide gels and identified by fluorography. The analytical results indicated the presence in good yield of a protein ($M_r$=75,000) indistinguishable from authentic glycosylated haemagglutinin (Elder *et al.* (1979) Virology *95*: 343—350). Synthesis of haemagglutinin occurred both at 37°C and to a lesser extent at 42°C. No such specific polypeptides were observed using any other transfecting DNA than of PR84 nor in immune complexes other than with anti-PR 8 antisera.

The details of the construction and expression of the eukaryotic expression vectors are now set forth.

A. Construction of plasmid 520

Fifty µg of plasmid pRV15 were digested first with 50 units of *Sma*I for 2 hours at 37°C and subsequently with 50 units of *Sal*I for 2 hours at 37°C (buffers as recommended by New England Biolabs). The digest was then electrophoresed on a 0.9% agarose gel. The region containing the 7 kb lac fragment was cut out of the gel, and the DNA was recovered by electroelution (200 v, 6 hours).

DNA in the eluate was collected by ethanol precipitation. The fragment was then dried and resuspended in 200 µl of TE buffer, phenol- and ether-extracted twice, and then passed through a small Sephadex® G75 column as described before. The DNA in the column eluate was collected by ethanol precipitation.

Ten µg of plasmid pSVod were digested with 10 units of *Bam*HI for 2 hours at 37°C. The digested DNA was purified by 3 phenol-extractions, 3 ether-extractions and 2 ethanol precipitations. The dried DNA was then resuspended in TE buffer and incubated with several units of DNA polymerase Klenow fragment in the presence of 0.5 mM deoxyadenosine triphosphate (dATP), deoxycytidine triphosphate (dCTP), deoxyguanosine triphosphate (dGTP) and deoxythymidine triphosphate (dTTP) at 6—7°C for one hour. The DNA was purified again by repeated phenol- and ether-extraction and ethanol precipitation. The purified DNA was then digested with 10 units of *Sal*I for 2 hours at 37°C. The digestion products were purified again as described above. The 7 kbp pRV15 *Sma*I/*Sal*I fragment and the above described pSVod fragments were then incubated overnight (in a molar ratio of 10:1) with an excess of T4 DNA ligase at 14°C. This ligation mixture was used to transform *E. coli* MC 1061. *Sma*I and *Bam*HI were included in the ligation mixture. Transformants were plated on LB plates containing 10 µg/ml ampicillin and 40 µg/ml Xgal. Blue transformants were isolated DNA from such recombinants was prepared as described by Schedl *et al.* (1978) Cell *14*: 921—929. Recombinants were identified by restriction analyses.

B. Construction of plasmid PR81

Fifty µg of plasmid A/PR 8/34 Clone No. 4.76 obtained from Prof. G. Brownlee, University of Oxford, U.K. were digested with 30 units of *Hin*dIII and *Bam*HI for 2 hours at 37°C. The digest was electrophoresed on a 0.85% agarose gel. The *Hin*dIII—*Bam*HI haemagglutinin gene fragment was purified by electroelution as described above. Aliquots of this DNA were nick translated to serve as probes for identifying PR81 and similar recombinants by Grunstein colony hybridization (specific radioactivity of probes about $10^8$ cpm/µg).

Fifty µg of A/PR 8/34/4.76 were digested with 50 units of *Bam*HI for 2 hours at 37°C. The DNA was phenol-extracted three times, ether-extracted and ethanol-precipitated. The dried DNA was then resuspended in 25 µl of TE buffer and incubated with several units of DNA polymerase Klenow fragment and 0.3 mM of all four deoxynucleotide triphosphates in a total volume of 100 µl for 90 minutes at 6—7°C. The DNA was then purified by phenol- and ether-extractions as above. Following ethanol precipitation, the DNA was dried and then resuspended in 25 µl of TE buffer. It was digested subsequently with 54 units of *Hin*dIII for one hour at 37°C. The digested DNA was repurified by phenol-extraction.

Ten µg of plasmid pSVod (Mellon *et al.* (1981) Cell *27*: 279—288) were digested with 30 units of *Sal*I for one hour at 37°C. The DNA was purified by phenol-extraction as above and was then incubated with several units of Klenow fragment in 100 µl in the presence of 0.5 mM, dATP, dCTP, dGTP and dTTP for one hour at 37°C. Following repurification by phenol-extraction, the DNA was digested further with 25 units of *Hind*III for one hour at 37°C and repurified by phenol extraction.

An A/PR 8/34 digest (1.5 µg) and a pSVod digest (2 µg) were incubated overnight at 14°C with an excess of T4 DNA ligase in a total volume of 30 µl. Several units of *Bam*HI and *Sal*I were included in the ligation mixture. The ligated DNA was purified by phenol extraction as described above. It was then digested with 6 units of *Bam*HI for one hour at 37°C, followed by incubation with 12 units of *Sal*I for one hour at 37°C. Aliquots of this reaction mixture were used to transform CaCl$_2$-treated *E. coli* C 600. Transformants were isolated on LB plates containing 10 µg/ml ampicillin. About 350 clones were purified and analyzed by colony hybridization using the nick translated *Hind*III—*Bam*HI haemagglutinin gene fragment described above as probe. Approximately 80—90% of the clones were found to contain a haemagglutinin gene insert.

Seven of the positive clones were grown up, and DNA was prepared from them. These DNAs were compared with the original clones A/PR 8/34 and pSVod by restriction digestion and electrophoresis on a 0.9% agarose gel.

Predicted fragment sizes

| Digestion with *Eco*RI | |
|---|---|
| Recombinants | 3.2 kbp, 1.5—1.6 kbp |
| A/PR 8/34 | 3.8 kbp, 1.3 kbp |
| pSVod | 3.3 kbp |

| Digestion with *Hind*III and *Sal*I | |
|---|---|
| Recombinants | 4.5 kbp |
| A/PR 8/34 | 3.3 kbp, 1.7—1.8 kbp |
| pSVod | 2.7 kbp, 0.6—0.7 kbp |

Five out of the seven recombinants that were analyzed showed the expected digestion pattern. PR81 is one of these five clones.

PR81 DNA was prepared as described by the method of Schedl *et al.* (1978) Cell *14*: 921—929 and was analyzed further by restriction enzyme digestion.

### C. Construction of plasmid PR84

p520 DNA (75 µg) was digested with 25 units of *Pvu*II for one hour at 37°C and subsequently with 20 units of *Nco*I for 2 hours at 37°C. The digest was electrophoresed on a 1.2% low melting agarose gel containing 0.05 µg/ml EtBr. The region including the two 1 kbp *Nco*I/*Pvu*II fragments (one of them is a hsp 70 gene promoter fragment) was cut out of the gel. The agarose piece and 10 volumes of TE buffer were heated to 65°C for 10 minutes, and the DNA was extracted subsequently with an equal volume of phenol. After one additional phenol/chloroform and 3 ether extractions, the DNA was precipitated two times with ethanol.

PR81 DNA (10 µg) was digested with 15 units of *Hind*III for one hour at 37°C. Following purification by phenol extraction the DNA was incubated with several units of Klenow fragment and 0.5 mM of all four deoxyribonucleotide triphosphates for one hour at 6—7°C. After repurification, the DNA was digested with 10 units of *Nco*I for 90 minutes at 37°C and was repurified by phenol extraction.

Aliquots (0.25 µg) of the p520 1 kbp *Nco*I/*Pvu*II fragments and aliquots (0.05—0.1 µg) of the PR81 fragments were incubated overnight at 14°C with an excess of T4 DNA ligase. This mixture was used to transform *E. coli* C 600. About 600 ampicillin-resistant transformants were isolated and examined by Grunstein colony hybridization using a nick translated p51 *Sau*3A/*Xho*I promoter fragment as a probe (approximately 10$^8$ cpm/µg) (Fig. 11*b*).

Ten positives were grown up and DNA was prepared from these clones. Aliquots of their DNAs were digested with *Xho*I and were analyzed on 5% polyacrylamide gels.

The presence and sizes of *Xho*I fragments provided good evidence for the presence in the recombinant DNAs of both the hsp 70 kdal gene promoter and the haemagglutinin gene. Four out of the 10 clones tested showed the expected restriction pattern (two fragments: one large, about 5 kbp, and one of 400 bp) (Figs. 11*c* and *d*).

Additional digestions with *Nco*I/*Xho*I and *Xba*I/*Nco*I confirmed our initial analysis. PR84 is one of the four plasmids which have the correct structure.

### D. Expression experiments using plasmid PR84

COS-I cells (Gluzman (1981) Cell *23*: 175—182) were maintained in Dulbecco's MEM with 10% new-born calf serum and were subcultured one day prior to transfection to give cultures containing about 10$^6$ cells per 6 cm diameter petri dish after a further one day of culture. Transfection of cells was performed · as follows:

The medium was removed from cell cultures at room temperature and the cultures were washed twice with 5 ml of phosphate-buffered saline (PBS). Subsequently one ml of the following preparations per dish were added.

1. 1 ml of Dulbecco's MEM with no serum, but containing DEAE Dextran (500 µg) and chloroquine (200 µg).
2. As 1, but containing in addition, 10 µg of plasmid 520 DNA
3. As 1, but containing in addition, 10 µg of plasmid PR84 DNA.

Transfections were performed on 5 dishes of cells for each of the three conditions stated above.

Cell cultures were maintained at room temperature for 30 minutes with occasional gentle rocking to distribute the transfection solution over the entire cell culture. After removal of the transfection solution, 5 ml per dish of Dulbecco's MEM with 10% serum was added. Cell cultures were further incubated at 37°C for 36 hours. Half of the two cultures were incubated at 42°C for the last 4 hours of this period. After this period, the medium was removed and cultures were washed once with a Dulbecco's MEM containing 3 mg/l of L-methionine and 1% new-born calf serum. Cell cultures were labelled by addition of 1 ml of this same low methionine medium per dish but in the presence of $^{35}$S-Methionine (50 µCi/ml, 968 Ci/mmol). Cultures were incubated for one hour at either 37°C or 42°C in the case of the heat-shock samples. After this period of labelling, the $^{35}$S-Methionine medium was removed, the cultures washed three times with ice-cold PBS, and cells were scraped off in 1 ml of NET buffer (0.1 M NaCl, 0.01 M Tris-HCl pH 7.8, 0.001 M EDTA, 0.5% NP 40). After vigorous pipetting to break down the outer cell membranes, nuclei and cellular debris were removed by centrifugation at 5000×g for 5 minutes at 4°C. The cytoplasmic supernatants were removed and aliquots were taken four immune precipitation as follows:

Aliquots (500 µl) of cytoplasmic extracts were kept on ice, 5 µl of specific antibodies were added and the solutions were mixed gently at 4°C for two hours. The antibodies used were the following: Normal rabbit antiserum, antiserum raised against purified *E. coli* β-galactosidase, and antiserum raised against PR8 influenza virus obtained from Dr. J. Shekel, MRC Laboratories, Mill Hill, London, U.K. Anti HA antisera was preadsorbed for 30 minutes in NET buffer using cytoplasmic extracts of unlabelled COS cells prior to use for immune precipitation of labelled samples.

Antigen-antixody complexes were isolated by the addition of 50 µl of a 50% suspension of protein A-Sepharose® (Pharmacia), continuing the shaking at 4°C for one hour, and recovering the bound complexes by centrifugation at 15,000×g for one minute. The complexes on protein A-Sepharose® were washed and centrifuged four times using 1 ml of NET and three times using 1 ml of RIPA buffer (0.15 M NaCl, 0.05 M Tris-HCl pH 7.5, 0.02 M EDTA, 1 M Urea, 1% Triton×100, 1% sodium deoxycholate). The final Sepharose pellets were boiled for 3 minutes in 100 µl of sample buffer (Laemmli (1970) Nature *227*: 680—685). After centrifugation at 15,000×g for 1 minute, the samples were subjected to electrophoresis on 10% polyacrylamide gels as indicated by Laemmli (1970) *supra.*

In the case of cell cultures transfected with plasmid PR84 DNA and in which the labelled cytoplasmic extracts were immune precipitated with antiserum raised against PR8 influenza virus, the gel analysis by fluorography (Laskey and Mills (1975) Eur. J. Biochem. *56*: 335—341) clearly indicated the presence of a protein which is indistinguishable in size ($M_r$=75,000) from authentic glycosylated haemagglutinin (Elder *et al.* (1979) Virology *95*: 343—350). Some possible processing to give polypeptides of $M_r$ approximately 42,000 and 36,000 was observed in some of these experiments. Synthesis of haemagglutinin occurred both when labelling was performed at 37°C and to a lesser extent at 42°C. No such specific polypeptides were observed using any other transfecting DNA than PR84, nor in immune precipitations other than with anti PR8 antisera.

In a second series of similar transfection experiments, COS cells transfected with PR84 were subjected to a period of heat-shock of five hours at 42°C and were subsequently labelled with $^{35}$S-Methionine as described above but at 37°C for 14 hrs. Subsequent immunoprecipitations with anti HA antisera indicated a larger quantity of HA product than in non heat-treated samples labelled under similar conditions.

These results suggest that the *Drosophila* heat-shock promoter functions better at the heat shock temperature in monkey cells than at the 37°C control temperature. Some activity of this promoter at 37°C is perhaps to be expected as we have previously noted the use of 37°C as the heat-shock temperature for *Drosophila* cells. The improved synthesis of HA when cells are returned to 37°C after a period of heat-shock suggests that the translational control of the *Drosophila* heat-shock promoter fragment, in particular the *Drosophilla* ribosome hinding site is non-functional or poorly functional in monkey cells at least at the higher temperature. Thus it is possible that although promotion of transcription occurs at 42°C using the *Drosophila* DNA sequences, ribosome binding and initiation of translation may occur using the HA ribosome hinding site, optimally at 37°C.

According to the present invention, expression vectors are provided which yield efficient transformation of eukaryotic hosts and high levels of expression within the host. In particular, the transcriptional/translational control region of a 70 kdal heat-shock protein has been isolated and joined to a replication system derived from a simian virus. A human influenza virus haemagglutinin gene was inserted into the resulting vector under the control of the said control region, and the resulting plasmid used to transfect a mammalian cell culture and apparently glycosylated haemagglutinin was produced.

Construction of plasmid p629

A further *Drosophila* control element/HA gene construction was made to extend the utility of plasmid constructions such as PR84. The HA gene being a viral gene is rather specialized as a model for any eucaryotic gene with its 3' end signals, and its RNA leader sequence, hence the PR84 construction contains both an HA RNA leader and a *Drosophila* RNA leader.

As a more general example of the construction of a gene expression unit using heat-shock control elements plasmid p629 was constructed. This plasmid contained in addition to the *Drosphila* heat-shock promoter the entire heat-shock RNA leader region, but was attached to a HA gene lacking the first 40 amino acid codons. For convenience, a plasmid p622b (a gift and personal communication of R. Voellmy) was employed; this plasmid contains a 450 bp *XhoI/BglII* fragment carrying the *Drosophila* hsp 70 promoter and RNA leader sequence (represented by the *XhoI/Sau*3A fragment of plasmid pRV15 described earlier). p622b is a derivative of the COS cell vector pSVod. The sequence around the *BglII* site is given below:

$$\text{...hsp 70—ATCGATCCAGATCTCGAG...HA gene...}$$

$$\uparrow \leftarrow\text{450 bp}\rightarrow \qquad \qquad \uparrow$$
$$Xho\text{I} \qquad \qquad Bg\text{/II} \quad Xho\text{I} \qquad Bam\text{HI}$$

Experimental construction of p629

15 μg of p622b were digested with 12 units (per 15 min) of *BglII*, and 15 μg of PR 8/ms/34 with 15 units of *XhoI*. Digests were for 2 hrs at 37°C. The DNAs were then extracted three times with phenol, three times with ether and precipitated two times with EtOH. Both DNAs were resuspended in 20 μl of TE buffer and ends were filled in a reaction involving several units of DNA polymerase (Klenow fragment) and 0.5 mM of each of the four dNTPS (total volume: 100 μl; reaction at 6°C for 2 hrs). Both DNAs were again phenol-extracted and purified as above and again dissolved in 20 μl of TE buffer. They were then digested individually with 15 units of *Bam*HI (total volume 100 μl; 37°C for 2 hrs). Following phenol extraction the DNAs were collected by EtOH precipitation and dissolved in 15 μl of TE buffer. Different sets of ligations containing 1 μl of 622b digest and 1—5 μl of PR 8/ms/34 digest in a total volume of 25 μl were carried out (enzyme: 1—2 μl, T4 DNA ligase, New England Biolabs) incubated overnight at 14°. The ligase was inactivated at 65°C for 10 min and the samples were then digested with several units of *Cla*I and *Sal*I successively, using the conditions suggested by New England Biolabs) for 2 hrs at 37°C in volumes of 50 μl. The DNA mixtures were then used to transform *E. coli* MC1061.

About 300 individual transformants were characterized further using the Grunstein colony hybridization assay. Two separate sets of hybridizations were carried out: probes used were: a) the 450 bp *Xho—BglII* fragment from p622b and b) 1775 bp *HindIII/Bam*HI gene fragment from PR8.4.76. Fragments had been separated in low melting agarose gels and were subsequently eluted from these gels. They were then labelled by nick translation ($10^7$—$10^8$ cpm/μg; $10^6$ cpm/filter). 8 colonies that hybridized to both probes were selected, (see Figs. 12$c$ and $d$) and DNA was prepared from them using the mini-DNA preparation procedure described previously. The DNAs were digested with *XhoI* or with *BglIII/Eco*RI and the digests were analyzed on 5% polyacrylamide gels. *XhoI* was expected to yield two fragments with sizes of 450 bp and of about 5kb. The *BglII/RI* digest should give fragments of 1130 bps, 1100 bps and of about 2.6 kb. Three of the eight clones tested had the correct restriction patterns (see Figs. 12$a$ and $b$).

Expression experiments using plasmid p629

Transfection of COS I cells was performed in a manner analogous to that described for the expression experiments using plasmid PR84. Immune precipitations performed as described previously, precipitated one major and a number of minor polypeptides of M.Wt around 75,000 daltons. When $^{35}$S-Methionine labelling was performed in the presence of 1 μg/ml tunicamycin, one major band ($M_r$=75,000) alone was observed, suggesting that the minor bands were the result of glycosylation. Synthesis of HA protein was greater during a labelling period of 14 hrs at 37°C following a period of heat-shock than in parallel labelling periods in the absence of heat-shock.

Although the foregoing invention has been described in some detail, by way of illustration and example for purposes of clarity and understanding, it will be obvious that certain changes and modifications may be practiced within the scope of the appended claims.

The cultures of *E. Coli* containing plasmids pRV-15, PR-84 and 629, respectively, cited in present disclosure have been registered on February 2, 1984 under Nos. 39597, 39596, 39598 at the American Type Culture Collection, 12301 Parklawn Drive, Rockville, Maryland 20852 U.S.A.

**Claims**

1. A "gene expression unit—host cell combination system" wherein the "gene expression unit" comprises (a) an inducible expression control region of 650 b.p. or less upstream of ATG of the coding region derived from a eucaryotic heat-shock protein gene; this region being functionally linked to a structural gene (b) whose expression products are of interest and wherein the "host cell system" comports cells transformed with said gene expression unit which are either from bacterial origin or from at least one species similar to those from which the said expression control region was isolated, characterized in that said products of interest expressed by said structural gene are products of translation.

2. A "gene expression unit—host cell system" as in claim 1, wherein the structural gene of interest is inserted into at least one site of the gene expression unit.

3. A "gene expression unit—host cell system" as in claim 2, wherein the heat-shock gene control region is derived from the genomic DNA of *Drosophila melanogaster*.

4. A "gene expression unit—host cell system" as in claim 3, wherein the heat-shock gene control region is derived from any one of the genes encoding the 70 kilo-dalton heat-shock proteins of *Drosophila melanogaster*.

5. A "gene expression unit—host cell system" as in claim 1, wherein at least part of said functional heat-shock gene control region is of synthetic origin.

6. A "gene expression unit—host cell system" as in claim 1, in which said expression unit further comprises at least one selectable marker linked to the expression unit which allows for selection of transformed hosts.

7. A "gene expression unit—host cell system" as in claim 1, in which said expression unit further comprises a procaryotic replication system which allows propagation of the expression unit, and at least one antibiotic resistance gene.

8. A "gene expression unit—host cell system" as in claim 1, wherein said expression unit is linked to a fragment containing a eucaryotic extrachomosomal replication system.

9. A "gene expression unit—host cell system" as in claim 1, wherein a heat-shock promoter element is linked to a sequence generating a complete RNA of a structural gene of interest.

10. A "gene expression unit—host cell system" as in claim 1, wherein a eucaryotic heat-shock gene promoter-RNA leader segment is linked to the protein-coding region of a structural gene of interest.

11. A "gene expression unit—host cell system" as in claim 1, wherein a eucaryotic heat-shock gene control element is linked to at least part of the DNA-coding region of a structural gene of interest.

12. Plasmid pRV15 identified as ATCC accession number 39597, as a new composition of matter.

13. Plasmid PR84, identifed as ATCC accession number 39596, as a new composition of matter.

14. A method for preparing the gene expression—host cell combination of claim 1, comprising introducing the expression unit into host cells by cotransformation with a selectable marker.

15. A method as in claim 14, wherein the expression unit is introduced into host cells with an amplifiable gene.

16. A method as in claim 14, wherein the expression unit is introduced into host cells by transformation or transfection.

17. An expression vector comprising:
a eukaryotic extrachromosomal replication system;
an expression control region of 650 b.p. or less upstream of ATG of the coding region derived from a eukaryotic heat-shock protein gene; and
at least one gene under the transcriptional and translational control of the expression control region.

18. An expression vector as in claim 17, said vector being free of a translational start codon between the expression control region and the gene.

19. An expression vector as in claim 17, having a translational start codon upstream from the gene.

20. An expression vector as in claim 17, further comprising a prokaryotic replication system which allows stable maintenance in prokaryotic hosts.

21. An expression vector as in claim 17, further comprising at least one selectable marker which allows for selection of transformed hosts.

22. An expression vector comprising:
a eukaryotic extrachromosomal replication system;
a heat-shock gene control region substantially homologous to a 650 base pair sequence upstream of ATG of the coding region which controls transcription of the 70 kilodalton heat-shock protein in *Drosophila melanogaster*; and
at least one gene under the transcriptional and translational control of the heat-shock gene control region.

23. A DNA construct of less 15 kbp which includes an expression control region of 650 b.p. or less upstream of ATG of the coding region derived from a eukaryotic heat-shock gene and at least one gene under the transcriptional and translational control of the heat-shock gene control region.

24. A DNA construct as in claim 23, wherein the heat-shock gene encodes the 70 kilodalton heat-shock protein of *Drosophila melanogaster*.

25. A method for producing products of translation of competent genes, said method comprising:
joining a structural gene of interest to an expression control region of 650 b.p. or less upstream of ATG of the coding region derived from a eukaryotic heat-shock gene;
introducing said structural gene and control region into a eukaryotic host suitable for expression of the structural gene; and
growing said host, whereby said products of translational are produced upon applying heat-shock.

26. A method as in claim 25, wherein the structural gene and control region are joined together in an expression vector having an eukaryotic extrachromosomal replication system.

27. A method as in claim 25, wherein the structural gene and control region are introduced to the host by co-transformation with a selectable marker.

## EP 0 118 393 B1

28. The method of claim 25, in which said structural gene under control of the regulatory sequences of a heat-shock gene are inserted into a plasmid carrying a selectable marker.

29. A method as in claim 25, wherein the expression control region is derived from a heat-shock gene which encodes a 70 kilodalton heat-shock protein in *Drosophila melanogaster*.

30. A method as in claim 25, wherein the heat-shock gene is derived from a different eukaryotic species from the host.

31. A method as in claim 25, wherein the structural gene is a mammalian gene.

32. A method as in claim 31, wherein the host is a mammalian cell culture.

33. A method as in claim 30, wherein the structural gene is a human gene.

34. A method as in claim 33, wherein the host is a mammalian cell culture.

35. A method as in claim 24, wherein the host is a COS I cell culture.

## Patentansprüche

1. "Genexpressionseinheit-Wirtszellenkombinationssystem", bei dem die "Genexpressionseinheit"
   a) einen induzierbaren Expressionskontrollbereich von 650 b.p. oder weniger oberhalb von ATG des codierenden Bereiches, abgeleitet von einem eucariotischen Heatschockproteingen, umfaßt, wobei dieser Bereich funktionell mit einem strukturellen Gen verbunden ist,
   b) dessen Expressionsprodukte von Interesse sind und wobei das "Wirtszellensystem" Zellen aufweist, die mit der Genexpressionseinheit transformiert sind, die entweder bakteriellen Ursprungs oder von wenigstens einer Spezies ähnlich jenen sind, von denen der Expressionskontrollbereich isoliert worden ist, dadurch gekennzeichnet, daß die interessierenden Produkte, ausgedrückt durch das sturkturelle Gen, Translationsprodukte sind.

2. "Genexpressionseinheit-Wirtszellensystem" gemäß Anspruch 1, bei dem das interessierende, strukturelle Gen an wenigstens einer Stelle der Genexpressionseinheit eingefügt ist.

3. "Genexpressionseinheit-Wirtszellensystem" gemäß Anspruch 2, bei dem der Heatschockgenkontrollbereich vom genomischen DNA von Drosophila melanogaster abgeleitet ist.

4. "Genexpressionseinheit-Wirtszellensystem" gemäß Anspruch 3, bei dem der Heatschockgenkontrollbereich von irgendeinem der Gene abgeleitet ist, die die 70 kg Dalton-Heatschockproteine von Drosophila melanogaster codieren.

5. "Genexpressionseinheit-Wirtszellensystem" gemäß Anspruch 1, bei dem wenigstens ein Teil des funktionen Heatschockgenkontrollbereiches synthetischen Ursprunges ist.

6. "Genexpressionseinheit-Wirtszellensystem" gemäß Anspruch 1, bei dem die Expressionseinheit weiters wenigstens eine wählbare Markiersubstanz enthält, die mit der Expressionseinheit verbunden ist, was die Wahl der transformierten Wirte gestattet.

7. "Genexpressionseinheit-Wirtszellensystem" gemäß Anspruch 1, bei dem die Expressionseinheit weiters ein procaryotisches Vervielfältigungssystem aufweist, was die Vermehrung der Expressionseinheit gestattet, und wenigstens ein antibiotisches Widerstandsgen hat.

8. "Genexpressionseinheit-Wirtszellensystem" gemäß Anspruch 1, bei dem die Expressionseinheit mit einem Fragment verbunden ist, das ein eucaryotisches, extrachomosomales Verfielfältigungssystem enthält.

9. "Genexpressionseinheit-Wirtszellensystem" gemäß Anspruch 1, bei dem ein Heatschockpromotionselement mit einer Sequenz verbunden ist, die ein vollständiges RNA eines interessierenden, strukturellen Gens bildet.

10. "Genexpressionseinheit-Wirtszellensystem" gemäß Anspruch 1, bei dem ein eucaryotisches Heatschockgenpromotions-RNA-Leadersegment mit einer proteincodierenden Region das interessierenden, strukturellen Gens verbunden ist.

11. "Genexpressionseinheit-Wirtszellensystem" gemäß Anspruch 1, bei dem ein eucaryotisches Heatschockgenkontrollelement mit wenigstens einem Teil DNA-codierenden Region eines interessierenden, strukturellen Gens verbunden ist.

12. Plasmid pRV15, identifiziert als ATCC, Zugangsnummer 39597, als eine neue Zusammensetzung der Substanz.

13. Plasmid PR84, identifiziert als ATCC, Zugangsnummer 39596, als eine neue Zusammensetzung der Substanz.

14. Verfahren zur Herstellung einer Genexpression - Wirtszellenkombination des Anspruches 1, bei dem die Expressionseinheit in die Wirtszellen durch Contransformation mit einem wählbaren Markierer eingebracht wird.

15. Verfahren nach Anspruch 14, bei dem die Expressionseinheit in die Wirtszellen mit einem verstärkbaren Gen eingebracht wird.

16. Verfahren nach Anspruch 14, bei dem die Expressionseinheit in die Wirtszellen durch Transformation oder Transfektion eingebracht wird.

17. Expressionsvektor mit:
   einem eucaryotischen, extrachromosomalen Vervielfältigungssystem,
   einem Expressionskontrollbereich von 650 b.p. oder weniger oberhalb der ATG des codierenden Bereiches, der von einem eucaryotischen Heatschockproteingen abgeleitet ist und

19

wenigstens einem Gen unter der transkritionalen oder translationalen Kontrolle des Expressionskontrollbereiches.

18. Expressionsvektor nach Anspruch 17, wobei der Vektor frei von einem translationalen Startcodon zwischen dem Expressionskontrollbereich und dem Gen ist.

19. Expressionsvektor nach Anspruch 17 mit einem translationalen Startcodon oberhalb des Gens.

20. Expressionsvektor nach Anspruch 17, der weiters ein procaryotisches Vervielfältigungssystem besitzt, das die stabile Aufrechterhaltung in procaryotischen Wirten gestattet.

21. Expressionsvektor nach Anspruch 17, der weiters wenigstens einen wählbaren Markierer aufweist, der die Auswahl der transformierten Wirte gestattet.

22. Expressionsvektor mit:

einem eucaryotischen, extrachromosomalen Vervielfältigungssystem,

einem Heatschockgenkontrollbereich, der im wesentlichen homolog zu

einer 650-Basispaarsequenz oberhalb des ATG des codierenden Bereiches ist, der die Transkription des 70-Kilodalton-Heatschockproteins in Drosophila malanogaster kontrolliert und

wenigstens einem Gen unter der transkriptionalen oder translationalen Kontrolle des Heatschockgenkontrollbereiches.

23. Ein DNA-Gebilde von weniger als 15 kbp, das einen Expressionskontrollbereich von 650 b.p. oder weniger oberhalb von ATG des codierenden Bereiches besitzt, das vom eucaryotischen Heatschockgen abgeleitet ist und wenigstens ein Gen unter der transkriptionalen und translationalen Kontrolle des Heatschockgenkontrollbereiches hat.

24. Ein DNA-Gebilde nach Anspruch 23, bei dem das Heatschockgen das 70-Kilodaltonheatschockprotein von Drosophila melanogaster codiert.

25. Verfahren zur Herstellung von Translationsprodukten der zuständigen Gene, wobei das Verfahren umfaßt:

Zusammenfügen eines interessierenden, strukturellen Gens zu einem Expressionskontrollbereich von 650 b.p. oder weniger oberhalb von ATG des codierenden Bereiches, der von einem eucaryotischen Heatschockgen abgeleitet ist.

Einfügen des strukturellen Gens und des Kontrollbereiches in einen eucaryotischen Wirt, geeignet für die Expression des strukturellen Gens und

Wachsen lassen des Wirtes, wodurch die Translationsprodukte durch Aufbringen von Heatschock hergestellt werden.

26. Verfahren nach Anspruch 25, bei dem das strukturelle Gen und der Kontrollbereich in einem Expressionsvektor zusammengefügt sind, der ein eucaryotisches, extrachomosomales Vervielfältigungssystem hat.

27. Verfahren nach Anspruch 25, bei dem das strukturelle Gen und der Kontrollbereich in der Wirt durch Cotransformation mit einem wählbaren Markierer eingebracht werden.

28. Verfahren nach Anspruch 25, bei dem das strukturelle Gen unter Kontrolle der regelnden Sequenzen des Heatschockgens in ein Plasmid eingebracht wird, das einen wählbaren Markieren trägt.

29. Verfahren nach Anspruch 25, bei dem der Expressionskontrollbereich von einem Heatschockgen abgeleitet wird, das ein 70-Kilodalton-Heatschockprotein in Drosophila melanogaster codiert.

30. Verfahren nach Anspruch 25, bei dem das Heatschockgen von einem verschiedenen Eucaryotikspezies vom Wirt abgeleitet ist.

31. Verfahren nach Anspruch 25, bei dem das strukturelle Gen ein Säugetiergen ist.

32. Verfahren nach Anspruch 31, bei dem der Writ eine Säugetierzellkultur ist.

33. Verfahren nach Anspruch 30, bei dem das strukturelle Gen ein menschliches Gen ist.

34. Verfahren nach Anspruch 33, bei dem der Wirt eine Säugetierzellkultur ist.

35. Verfahren nach Anspruch 34, bei dem der Wirt eine COS I-Zellenkultur ist.

**Revendications**

1. Un système associant des "unités d'expression de gènes" avec des "cellules-hôte" dans lequel le terme "unité d'expression de gènes" comprend (a) une région d'expression inductible de 650 p.b. ou moins placée en amont du chaînon ATG de la région de codage dérivée d'un gène de protéine de choc thermique (protéine "heat shock") cette région étant fonctionnellement liée à un gène structurel (b) dont l'expression est recherchée, et dans lequel le système de "cellules-hôte" comporte des cellules transformées par ladite unité d'expression de gènes, cellules qui sont, soit d'origine bactérienne, soit provenant d'au moins une espèce similaire à celle dont ladite région de contrôle d'expression est issue, caractérisé en ce que lesdits produits recherchés sont des produits de traduction.

2. Un système associant des "unités d'expression de gènes" avec des "cellules-hôte" suivant la revendication 1, dans lequel le gène structurel concerné est inséré dans au moins un site de l'unité d'expression de gènes.

3. Un système associant des "unités d'expression de gènes" avec des "cellules-hôte" suivant la revendication 2, dans dans lequel la région de contrôle du gène de choc thermique dérive de l'ADN génomique de *Drosophila Melanogastes*.

4. Un système associant des "unités d'expression de gènes" avec des "cellules-hôte" suivant la

## EP 0 118 393 B1

revendication 3, dans lequel la région de contrôle du gène de choc thermique dérive d'un quelconque des gènes contenant l'information génétique relative aux protéines de choc thermique de 70 kilodalton de *Drosophila Melanogastes*.

5. Un système associant des "unités d'expression de gènes" avec des "cellules-hôte" suivant la revendication 1, dans lequel au moins une partie de la région de contrôle dudit gène fonctionnel de choc thermique est d'origine synthétique.

6. Un système associant des "unités d'expression de gènes" avec des "cellules-hôte" suivant la revendication 1, dans lequel l'unité d'expression comprend au moins un marqueur de sélection lié à l'unité d'expression de manière à permettre une sélection des cellules-hôtes transformées.

7. Un système associant des "unités d'expression de gènes" avec des "cellules-hôte" suivant la revendication 1, dans lequel l'unité d'expression comporte encore un système de réplication eukaryote permettant la propagation de l'unité d'expression, et au moins un gène de résistance à un antibiotique.

8. Un système associant des "unités d'expression de gènes" avec des "cellules-hôte" suivant la revendication 1, dans lequel l'unité d'expression est liée à un fragment contenant un système eukaryote de réplication extrachromosomique.

9. Un système associant des "unités d'expression de gènes" avec des "cellules-hôte" suivant la revendication 1, dans lequel un élément promoteur "heat-shock" est lié à une séquence engrendrant l'ARN complet du gène structurel concerné.

10. Un système associant des "unités d'expression de gènes" avec des "cellules-hôte" suivant la revendication 1, dans lequel un segment "ARN-leader" de promoteur heat-shock d'origine eukaryote est lié à la région de codage de la protéine du gène structurel concerné.

11. Un système associant des "unités d'expression de gènes" avec des "cellules-hôte" suivant la revendication 1, dans lequel un élément de contrôle d'un gène de choc thermique eukaryote est lié à au moins une partie de l'ADN de la région de codage du gène structurel concerné.

12. Le plasmide pRV15 identifié par le numéro d'enregistrement ATCC 39597 en tant que produit nouveau.

13. Le plasmide PR84 identifié par le numéro d'enregistrement ATCC 39596 en tant que produit nouveau.

14. Procédé pour préparer la combinaison "unité d'expression de gènes—cellules-hôte" de la revendication 1, suivant lequel on introduit l'unité d'expression dans les cellules-hôte par co-transformation avec un marqueur de sélection.

15. Procédé suivant la revendication 14, dans lequel on introduit dans les cellules-hôte l'unité d'expression associée à un gène amplifiable.

16. Procédé suivant la revendication 14, dans lequel on introduit l'unité d'expression dans les cellules-hôte par transformation du transfection.

17. Un vecteur d'expression cellulaire comprenant:
un système de réplication extrachromosomique eukaryote;
une région de contrôle d'expression de 650 p.b. ou moins en amont du chaînon ATG de la région de codage dérivée d'un gène eukaryote contenant l'information génétique relative à une protéine de choc thermique; et
au moins un gène soumis au contrôle, en transcription et en traduction, de la région de contrôle d'expression.

18. Un vecteur d'expression suivant la revendication 17, ledit vecteur étant privé du codon commandant le départ de la traduction situé entre la région de contrôle d'expression et le gène.

19. Un vecteur d'expression suivant la revendication 17, comportant un codon commandant le départ de la traduction placé en amont dudit gène.

20. Un vecteur d'expression suivant la revendication 17, comportant, en plus, un système de réplication prokaryote permettant une culture stable dans les cellules-hôte prokaryotes.

21. Un vecteur d'expression suivant la revendication 17, comportant, en outre, un marqueur de sélection permettant la sélection des cellules-hôte transformées.

22. Un vecteur d'expression cellulaire comprenant:
un système de réplication extrachromosomique eukaryote,
une région de contrôle d'expression du gène de choc thermique pratiquement homologue avec la séquence de 650 p.b. en amont du chaînon ATG de la région de codage, cette séquence contrôlant la transcription de la protéine heat-shock de 70 kilodalton de *Drosophila Melanogastes*; et
au moins un gène placé sous le contrôle, en transcription et en traduction, de la région de contrôle du gène de choc thermique.

23. Un ADN recombinant de moins de 15 k.p.b. comportant une région de contrôle d'expression de 650 p.b. ou moins située en amont du chaînon ATG de la région de codage d'un gène de choc thermique d'origine eukaryote, et au moins un gène placé sous le contrôle, en transcription et en traduction, de cette région de contrôle du gène de choc thermique.

24. Un ADN recombinant suivant la revendication 23, dans lequel le gène de choc thermique contient l'information génétique de la protéine de choc thermique de 70 kilodalton de *Drosophila Mélanogastes*.

25. Procédé pour obtenir des produits de traduction de gènes fonctionnels, suivant lequel:

on relie un gène structurel intéressant à une région de contrôle d'expression de 650 p.b. ou moins situé en amont du chaînon ATG de la région de codage dérivée d'un gène eukaryote de choc thermique;

on introduit ledit gène structurel et la région de contrôle dans des cellules-hôte eukaryotes permettant l'expression du gène structurel, et

on cùltive lesdites cellules-hôte, lesdits produits de traduction étant obtenus par application d'un choc thermique.

26. Procédé suivant la revendication 25, dans lequel le gène structurel et la région de contrôle sont réunis dans un vecteur d'expression présentant un système de réplication eukaryote extrachromosomique.

27. Procédé suivant la revendication 25, dans lequel le gène structurel et la région de contrôle sont introduits dans les cellules-hôte par co-transformation avec un marqueur de sélection.

28. Procédé suivant la revendication 25, dans lequel ledit gène structurel placé sous contrôle de séquences de régulation d'un gène de choc thermique est introduit dans un plasmide comportant un marqueur de sélection.

29. Procédé suivant la revendication 25, dans lequel la région de contrôle d'expression provient d'un gène de choc thermique contenant l'information génétique relative à une protéine de choc thermique de 70 kilodalton de *Drosophila Melanogastes.*

30. Procédé suivant la revendication 25, dans lequel le gène de choc thermique dérive d'une espèce de cellules eukaryotes différente de celle des cellules-hôte.

31. Procédé suivant la revendication 25, dans lequel le gène structurel est un gène de mammifère.

32. Procédé suivant la revendication 31, dans lequel les cellules-hôte sont une culture de cellules de mammifères.

33. Procédé suivant la revendication 30, dans lequel le gène structurel est un gène humain.

34. Procédé suivant la revendication 33, dans lequel les cellules-hôte sont une culture de cellules de mammifères.

35. Procédé suivant la revendication 34, dans lequel les cellules-hôte sont une culture de cellules COS I.

FIG. 1a

FIG. 1b

1

-570    -560    -550    -540    -530    -520    -510
GCTTTCCTCATATGTACATATGTATGTAAATATGTAAAATAAGTCGCAACTAAATTCTAATACATTTTTCAGAA

-500    -490    -480    -470    -460    -450    -440    -430
TCTTAAATTAATTTTATCGTATATTAAAACAGAAGAAAGTCCGTTAATAGTTGATTTCATTAACTAAAAGTAC

-420    -410    -400    -390    -380    -370    -360
AAAATAATCTTTAATACATATGCCGATCAGACATTTATTGGTTTAGAAGCGCAGTATTTTTTTGGCGGAATAC
                    Sau3A

-350    -340    -330    -320    -310    -300    -290
GCATAACAAAGCGCTTCGATTATCTTTAACATAAGTTATTTAAGCAGCCGTATTTATAAAGAAATTTCCAAA

-280    -270    -260    -250    -240    -230    -220
ATAAAGC ATATATATAAATAAA GAATATTCTAGAATCCCAAAACAAACCTGGTTATTGTGGTAGGTCAT
                        XbaI

-210    -200    -190    -180    -170    -160    -150
TTGTTTGGCAGAAAGAAAACTCGAGAAATTTCTCTGGCCGTTATTCGTTATTCTCTCTTTTCTTTTTGGGT
                    XhoI

-140    -130    -120    -110    -100    -90    -80
CTCTCCCTCTCTGCACTAATGCTCTCTCACTCTGTCACACAGTAAACGGCATACTGCTCTCGTTGGTTC

-70    -60    -50    -40    -30    -20    -10
CAGAGAGCGCGCCTCGAATGTTCGCGAAAAGAGCGCCGGAGTATAAATAGAGGCGCTTCGTCTACGG

1    10    20    30    40    50    60
AGCGACAATTCAATTCAAACAAGCAAAGTGAACACGTCGCTAAGCGAAAGCTAAGCAAATAAACAAG
              1a

70    80    90    100    110    120
CGCAGCTGAACAAGCTAAACAATCTGCAGTAAAGTGCAAGTTAAAGTGAATCAATTAAAAGTAACCAG
                  PstI

130    140    150    160    170    180    190
CAACCAAGTAAAT AAACTAA CAACTGCAACTACTGAAATCTGCCAAGAAGTAATTATTGAATACAAGA

200    210    220    230    240    250    260
AGAGAACTCTGAATACTTTCAACAAGTTACCGAGAAAGAAGAACTCACACACAATGCCTGCTATTGGA
                                    1b

270    280
ATCGATCTGGGCACCACC
Sau3A

# FIG. 2

FIG. 3

EP 0 118 393 B1

FIG. 4a

FIG. 4b

FIG. 5a

FIG. 5b

FIG. 5c

FIG. 6a

FIG. 6b

EP 0 118 393 B1

FIG. 7a

FIG. 7b

FIG. 7c

FIG. 7d

FIG. 7e

7

FIG. 8a

FIG. 8b

8

FIG. 8c

F.G. 8d

FIG. 9a

Eco RI
Hind III
20
Bam HI
Pst I
Eco RI
4.76 kb

pA/PR8/34/ms/4.76

20

pSVod

FIG. 9b

Eco RI
Hind III
Sal I
Eco RI

FIG. 9c

Eco RI
Nco I
Hind III
(Bam HI)
(Sal I)
10
Eco RI
II
20

pR8l

FIG. 9d

FIG. 9e

FIG. 9f

FIG. 10a

FIG. 10b

FIG. 10c

FIG. 10d

FIG. 10e

14

*Eco RI*
*Nco I*
*Hind III*
*(Bam HI)*
*(Sma I)*
*Xba I*
*Xho I*

1                    10              20
ATTCAATTCAAACAAGCAAA

| 250 | 346 | ~ 400 |

          30          40          50          60
GTGAACACGTCGCTAAGCGAAAGCTAAGCAAATAAACAAG

      65                1           10          20          30
CGCAG AGCTTCAG AGCAAAAGCAGGGGAAAATAAAAACAACCA
(Pvu II / Hind III)

                 40           50          60          70
AAATGAAGGCAAACCTACTGGTCCTGTTATGTGCACTTGC

                    80          90          100         110
AGCTGCAGATGCAGACACAATATGTATAGGCTACCATGCG
    Pst I

              120         130         140
AACAATTCAACCGACACTGTTGACACAGTACTCGAG ——————
                                     Xho I

                        1770                          *Eco RI*
——————————————————————— // ——————————————— pSVod ———————
                    (Bam HI / Sal I)

PR 84

# FIG. 10f

15

FIG. IIa

FIG. II b (I)

FIG. IIb (2)

*1 2 3 4 5 6 7 8 9 10 11*

*FIG. IIc*

*1 2 3 4 5 6 7 8 9*

*bps*

1078 — 1353
872
603

278
271 — 310
234
194

118

72

*FIG. IId*

FIG. 12a

FIG. 12b

FIG. 12c

FIG. 12d